(19) Europäisches Patentamt
European Patent Office
Office européen des brevets

(11) EP 4 471 013 A1

(12) **EUROPEAN PATENT APPLICATION**
published in accordance with Art. 153(4) EPC

(43) Date of publication:
**04.12.2024 Bulletin 2024/49**

(21) Application number: **23746265.0**

(22) Date of filing: **19.01.2023**

(51) International Patent Classification (IPC):
***C07D 217/04*** (2006.01) ***C07D 223/16*** (2006.01)
***C07D 215/06*** (2006.01) ***C07D 265/36*** (2006.01)
***C07D 267/14*** (2006.01) ***A61K 31/472*** (2006.01)
***A61K 31/55*** (2006.01) ***A61K 31/47*** (2006.01)
***A61K 31/538*** (2006.01) ***A61K 31/553*** (2006.01)
***A61P 25/00*** (2006.01)

(52) Cooperative Patent Classification (CPC):
**A61K 31/47; A61K 31/472; A61K 31/538; A61K 31/55; A61K 31/553; A61P 25/00; C07D 215/06; C07D 217/04; C07D 223/16; C07D 265/36; C07D 267/14**

(86) International application number:
**PCT/CN2023/073185**

(87) International publication number:
**WO 2023/143386 (03.08.2023 Gazette 2023/31)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **25.01.2022 CN 202210089806**

(71) Applicant: **Shanghai Zhimeng Biopharma, Inc.**
**Shanghai 201210 (CN)**

(72) Inventors:
- **LIANG, Bo**
  **Shanghai 201210 (CN)**
- **LIU, Gang**
  **Shanghai 201210 (CN)**
- **JIANG, Zhaojian**
  **Shanghai 201210 (CN)**
- **HUANG, Mengwei**
  **Shanghai 201210 (CN)**
- **CHEN, Huanming**
  **Shanghai 201210 (CN)**

(74) Representative: **Santoro, Sofia et al**
**Società Italiana Brevetti S.p.A.**
**Via Giosuè Carducci, 8**
**20123 Milano (IT)**

# (54) AROMATIC RING-FUSED HETEROCYCLIC RING COMPOUND AS POTASSIUM CHANNEL REGULATOR, AND PREPARATION THEREFOR AND USE THEREOF

(57) The present invention relates to an aromatic ring-fused heterocyclic ring compound as a potassium channel regulator, and the preparation therefor and the use thereof. Specifically, the compound of the present invention has a structure as represented by formula (I), wherein the definition of each group and substituent is as described in the description. Further disclosed in the present invention are a method for preparing the compound and the use thereof as a potassium channel regulator.

EP 4 471 013 A1

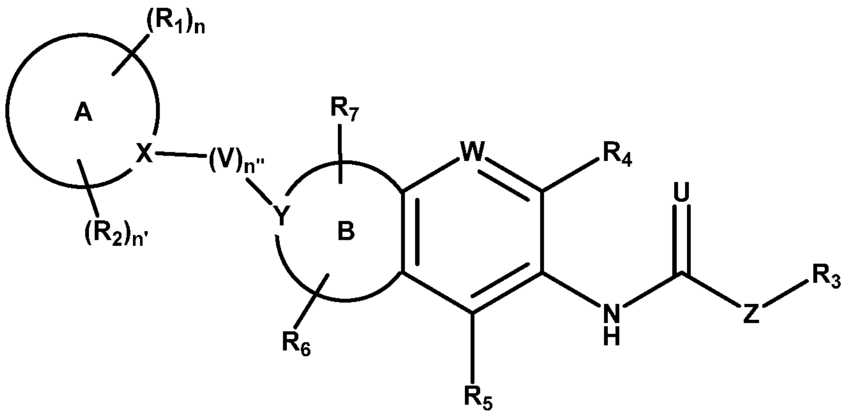

## Description

### Technical field

The present invention relates to biomedical field, specifically to an aromatic ring-fused heterocyclic ring compound as potassium channel regulator, and preparation therefor and use thereof.

### Background

Kv7 potassium channel is a class of voltage dependent potassium ion channels, characterized by low threshold activation, slow activation, and non deactivation. The Kv7 potassium channel has five family members (Kv7.1-Kv7.5), and all Kv7 potassium channel members have similar topological structures, i.e., four subunits form a functional channel, with each subunit containing six transmembrane segments (S1-S6). Among them, S4 is the voltage sensing region, which plays an important role in sensing membrane potential changes and controlling conformational changes; SS-S6 is the main component of the channel pore area and the main combination and action area of potassium channel opener. KV7.1 potassium channel is a non neuronal pathway distributed in peripheral tissues and expressed in the heart to mediate myocardial Iks, and the mutations of which can lead to Long Q-Tsyndrome. Kv7.2-Kv7.5 potassium channels are the basis of neuronal M currents, widely distributed in the nervous system, and have various physiological activities. Mutations in the Kv7.2 and Kv7.3 potassium channel genes can lead to various epilepsy phenotypes, such as benign familial neonatal convulsions (BFNC), which fully demonstrate the role of M currents in regulating neuronal excitability. Kv7.4 potassium channel is highly expressed in the outer hair cells of the cochlea and brainstem auditory nucleus, and its mutations may lead to hereditary hearing loss. Kv7.5 potassium channel is highly expressed in skeletal muscle and brain, and its mutations may lead to retinopathy. Many diseases such as epilepsy, anxiety, and deafness share the common feature of high membrane excitability, and Kv7 potassium channels, as the molecular basis for M-currents, can be opened by sensing changes in membrane potential, and upregulat inhibitory potassium currents, thereby controlling membrane excitability, making Kv7 potassium channels of great significance in pain and mental disorders represented by high neural excitability.

Retigabine is a drug used to treat epilepsy and has been approved for marketing in UK, Germany, and Denmark. Research has confirmed that the action of Ritegabine is related to voltage gated potassium ion channels (KCNQs), with its main mechanism of action being the regulation of M-type potassium currents by KCNQ2/3 channels.

Ritegabine (RTG) was the first Kv7 potassium channel opener launched in 2011 as an adjuvant therapy for adult partial seizure epilepsy. In addition to anti-epileptic effects, RTG can also be used to treat anxiety disorders, neuralgia, neurodegenerative diseases, etc. RTG can effectively reduce or prevent epileptic seizures in various epilepsy models. RTG has shown effective anti epileptic effects on both tonic seizures induced by the Maximum Electroshock (MES) model and clonic seizures induced by PTZ. In addition, RTG can also prevent epileptic seizures caused by N-methyl-D-aspartate (NMDA), penicillin, picrotoxin, kainic acid (KA), etc. The ignition model is suitable for screening various anti-epileptic drugs, and the effect of RTG on this model is stronger than on other models. Due to the extensive effects of RTG on all Kv7 potassium channel members and other channels, its selectivity is poor, making it potentially harmful. A large number of literatures have reported that RTG has a high incidence of adverse events related to the central nervous system, which can lead to dizziness, fatigue, aphasia, speech disorders, balance disorders and other adverse reactions, including kidney stones, urinary retention and other kidney and urinary system diseases, cardiac arrest, transient non persistent ventricular tachycardia and other heart related diseases, and can also lead to retinal discoloration, skin, nails and other blue/purple pigmentation.

### Summary

The purpose of the present invention is to provide a compound shown in formula I and preparation method therefor, and the use as potassium channel regulator.

In the first aspect of the present invention, provided is a compound of formula I, or a pharmaceutically acceptable salt thereof,

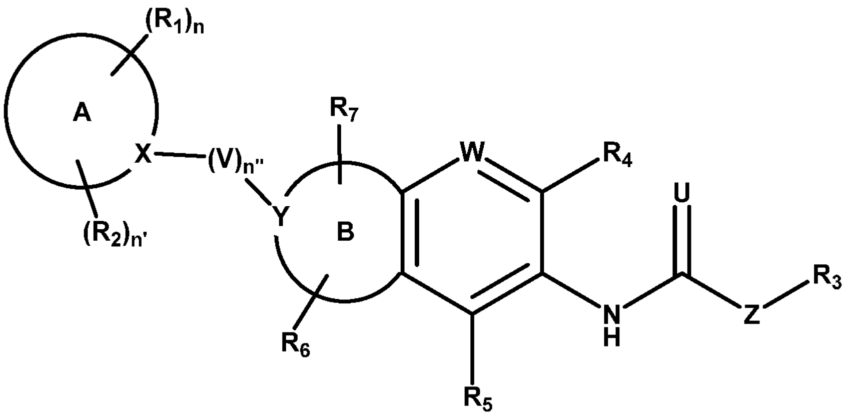

Formula I

wherein,

ring A is selected from the group consisting of: $C_{6-10}$ aryl, 4-7-membered heteroaryl containing 1-3 heteroatoms selected from N, O and S, saturated or unsaturated $C_{3-6}$ cyclic hydrocarbon group, and 3-10-membered heterocyclyl containing 1-3 heteroatoms selected from N, O and S;

each $R_1$ and $R_2$ is independently selected from the substituted or unsubstituted group consisting of: hydrogen, deuterium, halogen, cyano, -OH, -COOH, nitro, $C_{1-6}$ alkyl, $C_{3-6}$ cycloalkyl, $C_{1-6}$ alkoxy, $C_{3-6}$ cycloalkyloxy, $C_{2-6}$ alkenyl, $C_{2-6}$ alkynyl, saturated or unsaturated $C_{3-6}$ cyclic hydrocarbon group, 3-10-membered heterocyclyl containing 1-3 heteroatoms selected from N, O and S, $C_{6-10}$ aryl, 5-14-membered heteroaryl containing 1-3 heteroatoms selected from N, O and S, $C_{6-12}$ arylalkyl, $-N(R_1')(R_2')$, $-C(O)-R_1'$, $-C(O)-N(R_1')(R_2')$, $-C(O)-OR_1'$, $-N(R_1')-C(O)-R_2'$, $-S(O)_m-R_1'$, $-S(O)_m-N(R_1')(R_2')$, $-S(O)_m-OR_1'$, $-N(R_1')-S(O)_m-R_2'$, and the "substituted" refers to being substituted by one or more substituents selected from the group consisting of: halogen, $C_{1-6}$ alkyl, $C_{3-6}$ cycloalkyl, $C_{1-6}$ alkoxy, $C_{3-6}$ cycloalkyloxy, $C_{1-6}$ haloalkyl, $C_{3-6}$ halocycloalkyl, $C_{1-6}$ haloalkoxy, and $C_{3-6}$ halocycloalkyloxy;

n is selected from the group consisting of: 0, 1 and 2;

n' is selected from the group consisting of: 0, 1 and 2;

each $R_1'$ and $R_2'$ are independently selected from the group consisting of: hydrogen,

$C_{1-6}$ alkyl, $C_{3-6}$ cycloalkyl, or $R_1'$ and $R_2'$ together with the attached N-atom form a saturated or unsaturated 3-10-membered heterocyclyl containing 1-3 heteroatoms selected from N, O and S; and the above alkyl, cycloalkyl and heterocyclyl are optionally substituted by one or more substituents selected from the group consisting of: =O, halogen, $C_{1-6}$ alkyl and $C_{3-6}$ cycloalkyl;

m is selected from the group consisting of: 1 and 2;

X is selected from the group consisting of: C, CRs and N;

each $R_8$ is independently selected from the group consisting of: H, $C_{1-6}$ alkyl, $C_{3-6}$ cycloalkyl, $C_{2-6}$ alkenyl and $C_{2-6}$ alkynyl; and the above alkyl and cycloalkyl are optionally substituted by one or more substituents selected from the group consisting of: halogen, $C_{1-6}$ alkyl and $C_{3-6}$ cycloalkyl;

V is selected from the group consisting of: $-C(R_9)(R_{10})-$ and -N(Rs)-;

n" is selected from the group consisting of: 0 and 1;

$R_9$ and $R_{10}$ are independently selected from the group consisting of: hydrogen, halogen, and $C_{1-6}$ alkyl, or $R_9$ and $R_{10}$ together with the attached C-atom form a $C_{3-6}$ cycloalkyl;

ring B is selected from the group consisting of: saturated or unsaturated $C_{3-10}$ cyclic hydrocarbon group, and saturated or unsaturated 3-10 membered heterocyclyl containing 1-3 heteroatoms selected from N, O and S;

$R_6$ and $R_7$ are independently selected from the group consisting of: hydrogen, deuterium, halogen, $C_{1-6}$ alkyl, $C_{3-6}$ cycloalkyl, $C_{2-6}$ alkenyl, $C_{2-6}$ alkynyl, $C_{1-6}$ haloalkyl and $C_{3-6}$ halocycloalkyl;

Y is selected from the group consisting of: CRs and N;

W is selected from the group consisting of: $CR_{11}$ and N;

$R_{11}$ is selected from the group consisting of: hydrogen, deuterium, halogen, cyano, amino, $C_{1-6}$ alkyl, $C_{3-6}$ cycloalkyl, $C_{1-6}$ alkoxy, $C_{3-6}$ cycloalkyloxy, and $-N(R_1')(R_2')$; and the above alkyl, cycloalkyl, and alkoxy are optionally substituted by one or more substituents selected from the group consisting of: halogen, $C_{1-6}$ alkyl and $C_{3-6}$ cycloalkyl;

$R_4$ and $R_5$ are independently selected from the group consisting of: hydrogen, deuterium, halogen, cyano, amino, $C_{1-6}$ alkyl, $C_{3-6}$ cycloalkyl, $C_{1-6}$ alkoxy, $C_{3-6}$ cycloalkyloxy and $-N(R_1')(R_2')$; and the above alkyl, cycloalkyl, and alkoxy are optionally substituted by one or more substituents selected from the group consisting of: halogen, $C_{1-6}$ alkyl and $C_{3-6}$ cycloalkyl;

U is selected from the group consisting of: O, S and $N(R_1')$;

Z is selected from the group consisting of: O, $-(CH_2)_q-$ and $-N(R_1')-$;
q is selected from the group consisting of: 0, 1 and 2;
$R_3$ is selected from the group consisting of: $C_{1-6}$alkyl, $C_{3-6}$cycloalkyl, $C_{5-8}$ bridged cyclic group, adamantyl, $C_{6-10}$ aryl, 3-10-membered heteroaryl containing 1-3 heteroatoms selected from N, O and S, 4-8-membered heterocycloalkyl containing 1-3 heteroatoms selected from N, O and S, $C_{3-6}$ cycloalkenyl, $C_{2-6}$ alkenyl and $C_{2-6}$ alkynyl; and the above alkyl, cycloalkyl, bridged cyclic group, adamantyl, aryl, heteroaryl, heterocycloalkyl, cycloalkenyl, alkenyl, and alkynyl are optionally substituted by one or more substituents selected from the group consisting of: hydrogen, halogen, cyano, nitro, amino, hydroxyl, $C_{1-6}$ alkyl-CO-, $C_{1-6}$ alkyl, $C_{3-6}$ cycloalkyl, $C_{6-10}$ aryl, $C_{1-6}$ haloalkyl, $C_{1-6}$ alkoxy, $C_{1-6}$ alkylamino and $C_{1-6}$ haloalkoxy.

In another preferred embodiment,

ring A is selected from the group consisting of: $C_{6-10}$ aryl, and 4-7-membered heteroaryl containing 1-3 heteroatoms selected from N, O and S;
each $R_1$ and $R_2$ are independently selected from the substituted or unsubstituted group consisting of: hydrogen, deuterium, halogen, cyano, $C_{1-6}$ alkyl, $C_{3-6}$ cycloalkyl, $C_{1-6}$ alkoxy, $C_{3-6}$ cycloalkyloxy, $C_{2-6}$ alkenyl, $C_{2-6}$ alkynyl and $-N(R_1')(R_2')$, and the "substituted" refers to being substituted by one or more substituents selected from halogen;
n is selected from the group consisting of: 0, 1 and 2;
n' is selected from the group consisting of: 0, 1 and 2;
each $R_1'$ and $R_2'$ are independently selected from the group consisting of: hydrogen, $C_{1-6}$ alkyl and $C_{3-6}$ cycloalkyl; the above alkyl and cycloalkyl are optionally substituted by one or more substituents selected from halogen;
X is C;
V is $CH_2$;
n" is selected from the group consisting of: 0 and 1;
ring B is a saturated or unsaturated 3-10 membered heterocyclyl containing 1-3 heteroatoms selected from N, O and S;
$R_6$ and $R_7$ are independently selected from the group consisting of: hydrogen and deuterium;
YisN;
W is selected from the group consisting of: $CR_{11}$ and N;
$R_{11}$ is selected from the group consisting of: hydrogen, halogen and $C_{1-6}$alkyl; and the above alkyl is optionally substituted by one or more substituents selected from halogen;
$R_4$ and $R_5$ are independently selected from the group consisting of: hydrogen, halogen, cyano, amino, $C_{1-6}$ alkyl, $C_{3-6}$ cycloalkyl, $C_{1-6}$ alkoxy and $C_{3-6}$ cycloalkyloxy; and the above alkyl, cycloalkyl and alkoxy are optionally substituted by one or more substituents selected from halogen;
U is O;
Z is selected from the group consisting of: O and $CH_2$;
$R_3$ is selected from the group consisting of: $C_{1-6}$ alkyl, $C_{3-6}$ cycloalkyl, $C_{5-8}$ bridged cyclic group and $C_{2-6}$ alkynyl; and the above alkyl, cycloalkyl, bridged cyclic group, and alkynyl are optionally substituted by one or more substituents selected from the group consisting of: hydrogen, halogen, $C_{1-6}$ alkyl, $C_{3-6}$ cycloalkyl, $C_{1-6}$ haloalkyl, $C_{1-6}$ alkoxy and $C_{1-6}$ haloalkoxy.

In another preferred embodiment,

ring A is $C_{6-10}$ aryl;
each $R_1$ and $R_2$ are independently selected from the substituted or unsubstituted group consisting of: hydrogen, halogen, $C_{1-6}$ alkyl, $C_{3-6}$ cycloalkyl, $C_{1-6}$ alkoxy, $C_{3-6}$ cycloalkyloxy, $C_{2-6}$ alkynyl and $-N(R_1')(R_2')$, and the "substituted" refers to being substituted by one or more substituents selected from halogen;
n is selected from the group consisting of: 0, 1 and 2;
n' is selected from the group consisting of: 0, 1 and 2;
each $R_1'$ and $R_2'$ are independently selected from the group consisting of: hydrogen and $C_{1-6}$ alkyl;
X is C;
V is $CH_2$;
n" is selected from the group consisting of: 0 and 1;
ring B is a saturated or unsaturated 3-10-membered heterocyclyl containing 1-3 heteroatoms selected from N, O and S;
$R_6$ and $R_7$ are independently selected from the group consisting of: hydrogen and deuterium;
YisN;
W is CH;

$R_4$ and $R_5$ are independently selected from the group consisting of: halogen, $C_{1-6}$alkyl and $C_{1-6}$alkoxy; and the above alkyl and alkoxy are optionally substituted by one or more substituents selected from halogen;
U is O;
Z is $CH_2$;
$R_3$ is selected from the group consisting of: $C_{3-6}$ cycloalkyl and $C_{5-8}$ bridged cyclic group; and the above cycloalkyl and bridged cyclic group are optionally substituted by one or more substituents selected from the group consisting of: hydrogen, halogen, $C_{1-6}$alkyl and $C_{1-6}$haloalkyl.

In another preferred embodiment, ring B is selected from the group consisting of:

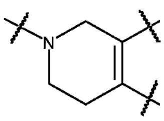, 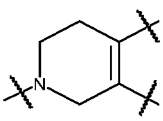, 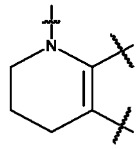, 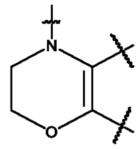, 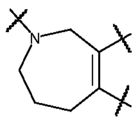, 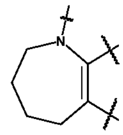, 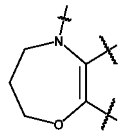, 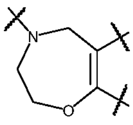, 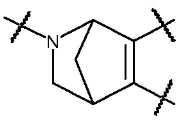, 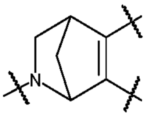, 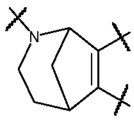, 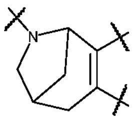, 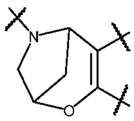, 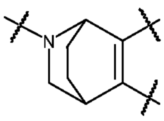, 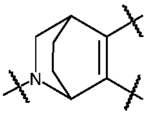, 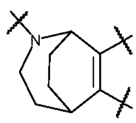, 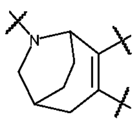 and 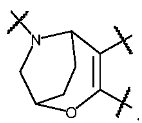.

In another preferred embodiment, $R_3$ is selected from the group consisting of: $C_{3-6}$ cycloalkyl and $C_{5-8}$ bridged cyclic group.

In another preferred embodiment, $R_3$ is selected from the group consisting of: $C_{3-6}$ cycloalkyl and $C_{5-8}$ bridged cyclic group, and the above cycloalkyl and bridged cyclic group are optionally substituted by one or more substituents selected from the group consisting of: halogen, $C_{1-6}$alkyl and $C_{1-6}$haloalkyl.

In another preferred embodiment, the compound does not include the following compounds:

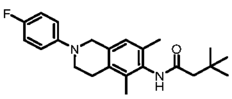 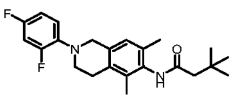 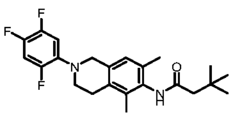 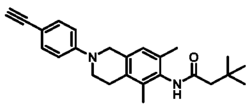 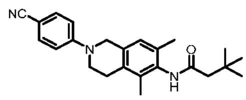

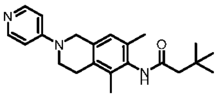 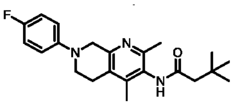 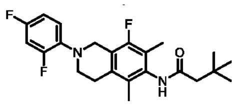 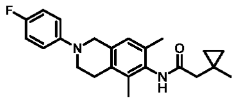 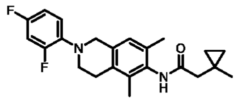

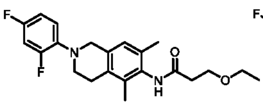 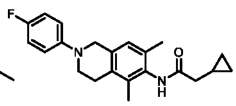 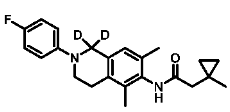 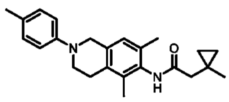 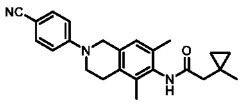

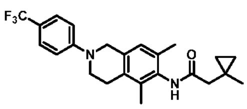 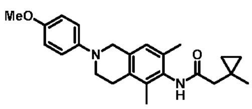 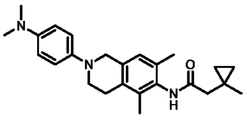 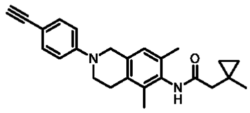 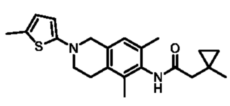

and

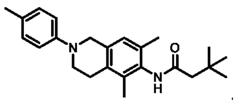

In another preferred embodiment,

ring A is phenyl;
each $R_1$ and $R_2$ are independently selected from the substituted or unsubstituted group consisting of: hydrogen, halogen, $C_{1-6}$ alkyl, $C_{1-6}$ alkoxy, $C_{2-6}$ alkynyl, and -N($R_1$')($R_2$'), and the "substituted" refers to being substituted by one or more substituents selected from halogen;
n is selected from the group consisting of: 0, 1 and 2;
n' is selected from the group consisting of: 0, 1 and 2;
each $R_1$' and $R_2$' are independently selected from the group consisting of: hydrogen and $C_{1-6}$ alkyl;
X is C;
n" is 0;
ring B is selected from the group consisting of:

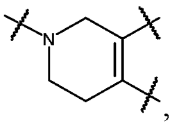, 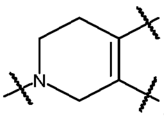, 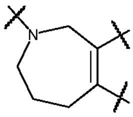,

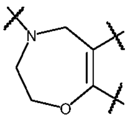, 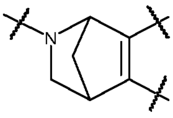, 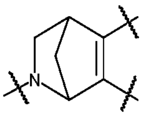, 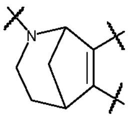, 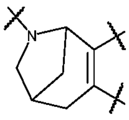, 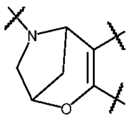, 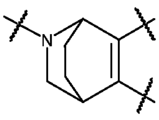,

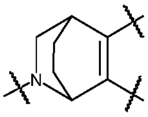, 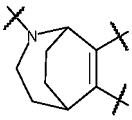, 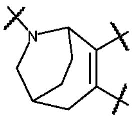 and 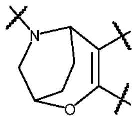;

$R_6$ and $R_7$ are independently selected from the group consisting of: hydrogen and deuterium;
W is CH;
$R_4$ and $R_5$ are independently selected from the group consisting of: halogen, $C_{1-6}$alkyl and $C_{1-6}$alkoxy; and the above alkyl and alkoxy are optionally substituted by one or more substituents selected from halogen;
U is O;
Z is $-CH_2-$;
$R_3$ is selected from the group consisting of: $C_{3-6}$ cycloalkyl and $C_{5-8}$ bridged cyclic group; and the above cycloalkyl and bridged cyclic group are optionally substituted by one or more substituents selected from the group consisting of: hydrogen, halogen, $C_{1-6}$alkyl and $C_{1-6}$haloalkyl.

In another preferred embodiment,

ring A is phenyl;
each $R_1$ and $R_2$ are independently selected from the substituted or unsubstituted group consisting of: hydrogen, halogen, $C_{1-6}$ alkyl, $C_{1-6}$ alkoxy, $C_{2-6}$ alkynyl, and -N($R_1$')($R_2$'), and the "substituted" refers to being substituted by one or more substituents selected from halogen;
n is selected from the group consisting of: 0, 1 and 2;
n' is selected from the group consisting of: 0, 1 and 2;
each $R_1$' and $R_2$' are independently selected from the group consisting of: hydrogen and $C_{1-6}$ alkyl;
X is C;
n" is 0;
ring B is selected from the group consisting of:

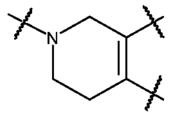,
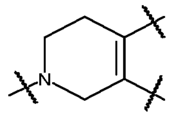

and

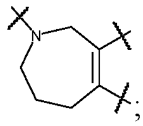;

$R_6$ and $R_7$ are independently selected from the group consisting of: hydrogen and deuterium;
W is CH;
$R_4$ and $R_5$ are independently selected from the group consisting of: halogen, $C_{1-6}$alkyl and $C_{1-6}$alkoxy; and the above alkyl and alkoxy are optionally substituted by one or more substituents selected from halogen;
U is O;
Z is $-CH_2-$;
$R_3$ is selected from the group consisting of: $C_{3-6}$ cycloalkyl and $C_{5-8}$ bridged cyclic group; and the above cycloalkyl and bridged cyclic group are optionally substituted by one or more substituents selected from the group consisting of: hydrogen, halogen, $C_{1-6}$alkyl and $C_{1-6}$haloalkyl.

In another preferred embodiment,

ring A is phenyl;
each $R_1$ and $R_2$ are independently selected from the substituted or unsubstituted group consisting of: hydrogen, halogen, $C_{1-6}$ alkyl, $C_{1-6}$ alkoxy, $C_{2-6}$ alkynyl, and $-N(R_1')(R_2')$, and the "substituted" refers to being substituted by one or more substituents selected from halogen;
n is selected from the group consisting of: 0, 1 and 2;
n' is selected from the group consisting of: 0, 1 and 2;
each $R_1'$ and $R_2'$ are independently selected from the group consisting of: hydrogen and $C_{1-6}$ alkyl;
X is C;
V is $-CH_2-$;
n" is 1;
ring B is selected from the group consisting of:

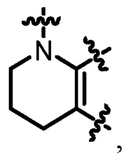,
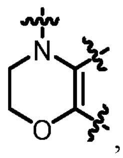,
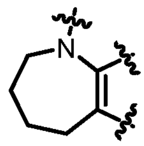

and

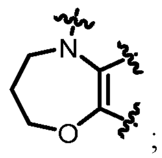;

$R_6$ and $R_7$ are independently selected from the group consisting of: hydrogen and deuterium;
W is CH;
$R_4$ and $R_5$ are independently selected from the group consisting of: halogen, $C_{1-6}$alkyl and $C_{1-6}$alkoxy; and the above alkyl and alkoxy are optionally substituted by one or more substituents selected from halogen;
U is O;
Z is $-CH_2-$;
$R_3$ is selected from the group consisting of: $C_{3-6}$ cycloalkyl and $C_{5-8}$ bridged cyclic group; and the above cycloalkyl and bridged cyclic group are optionally substituted by one or more substituents selected from the group consisting of:

hydrogen, halogen, $C_{1-6}$alkyl and $C_{1-6}$haloalkyl.

In another preferred embodiment, the compound is selected from the group consisting of:

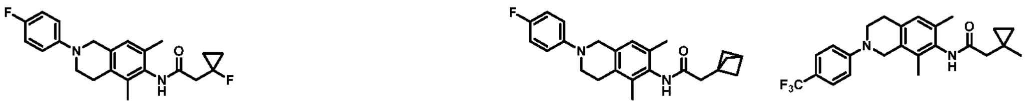

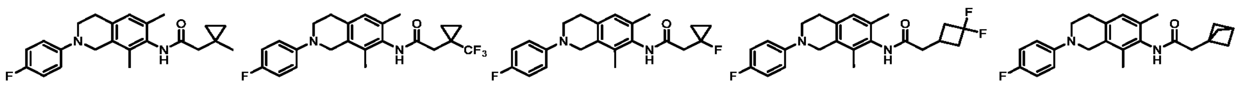

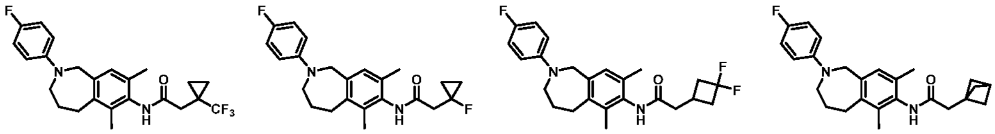

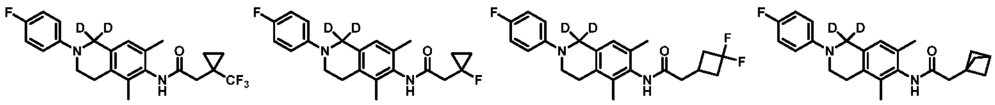

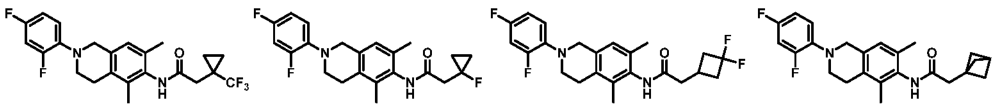

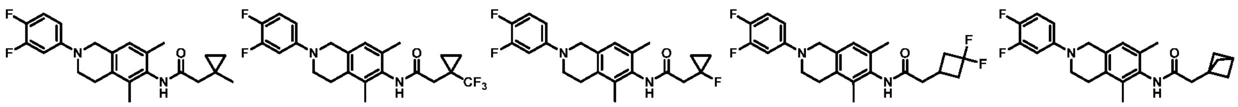

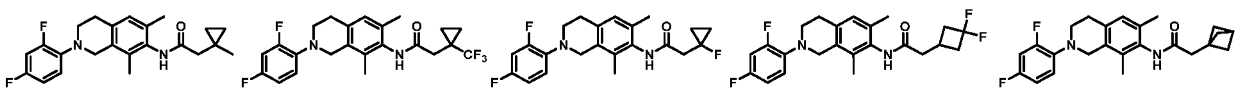

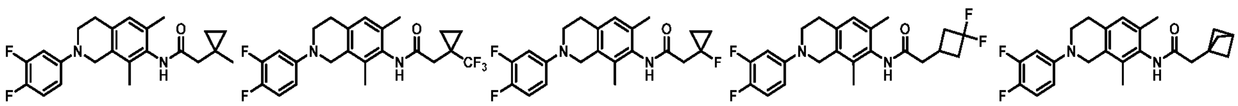

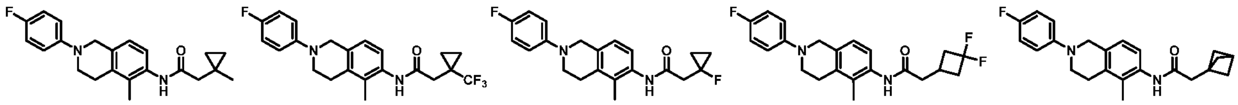

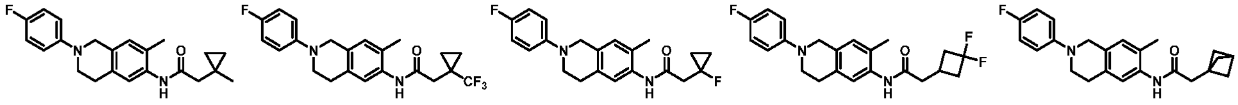

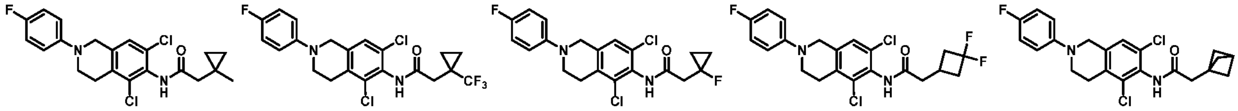

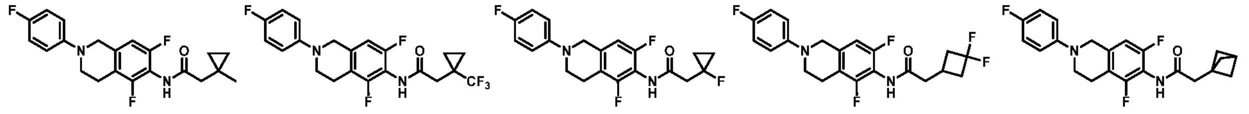

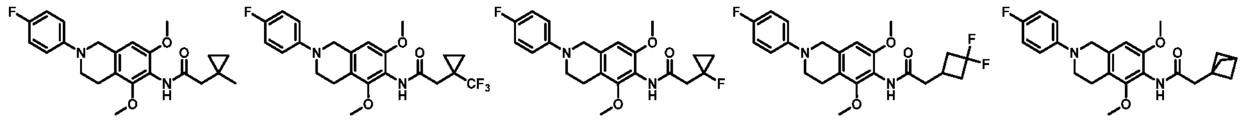

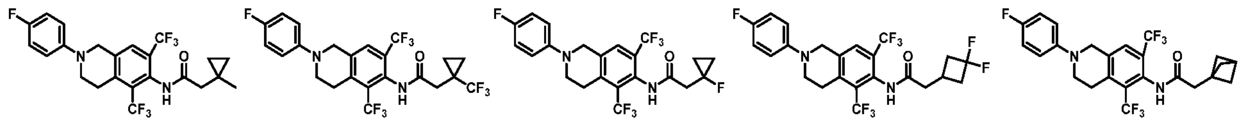

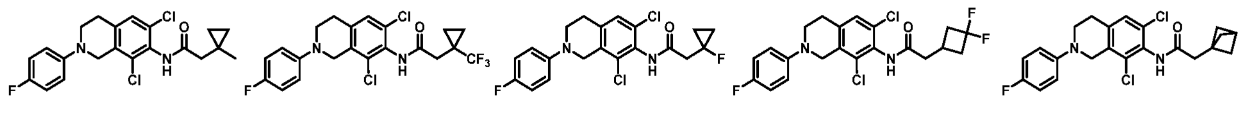

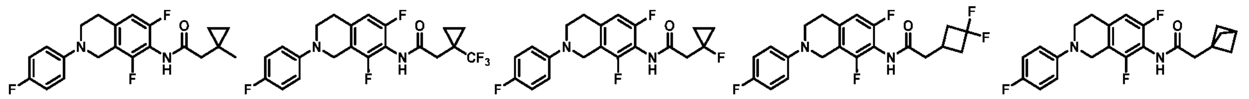

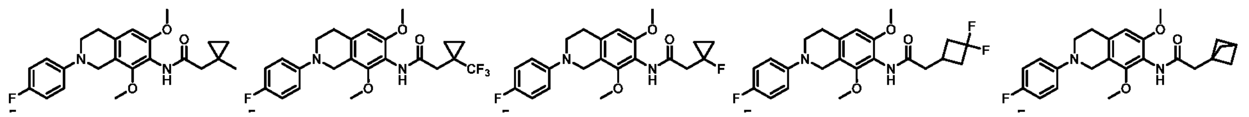

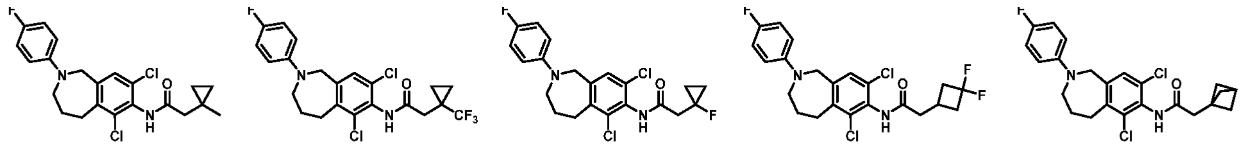

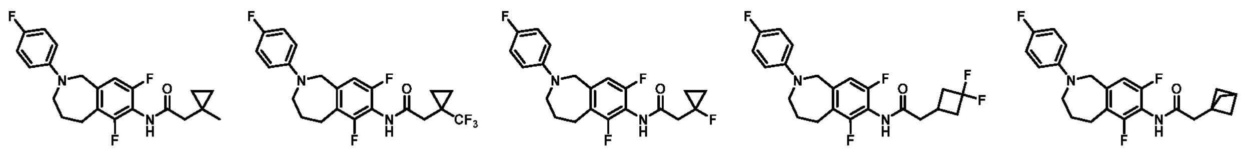

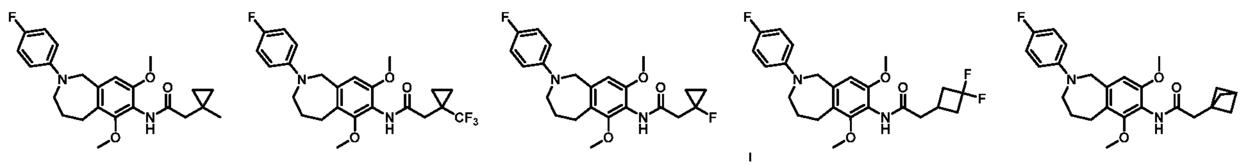

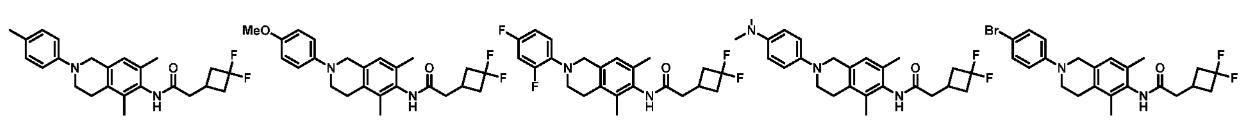

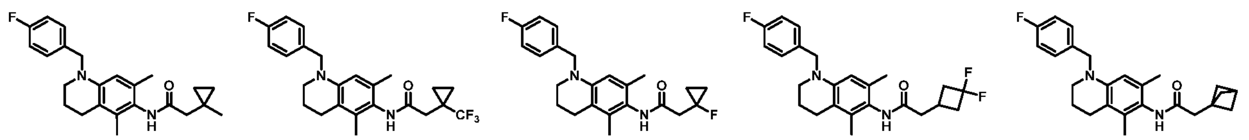

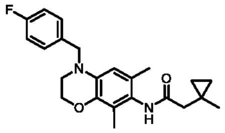 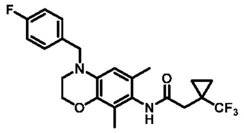 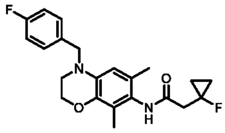 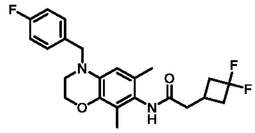 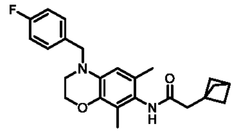

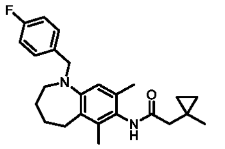 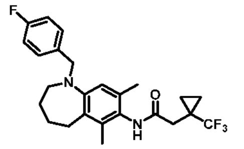 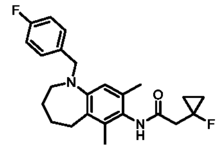 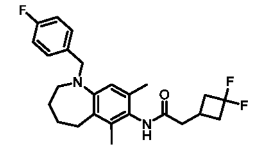 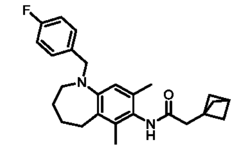

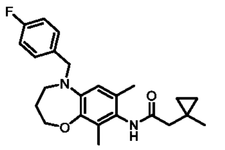 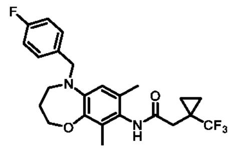 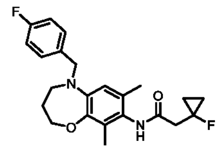 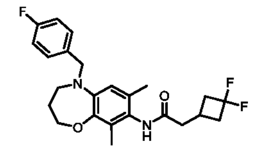 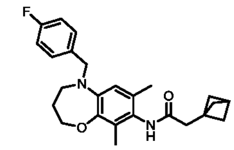

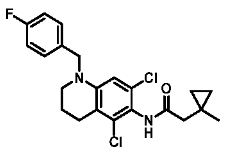 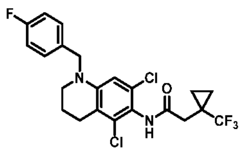 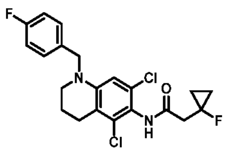 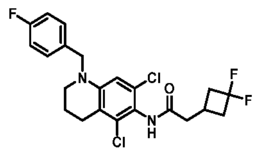 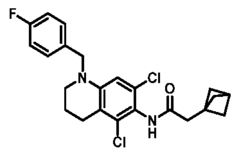

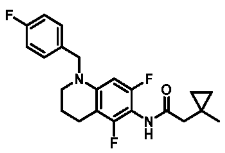 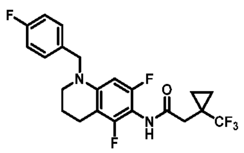 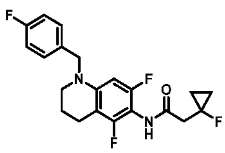 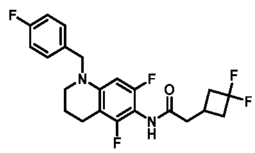 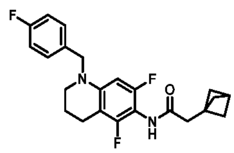

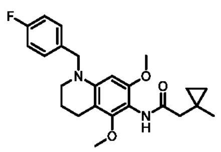 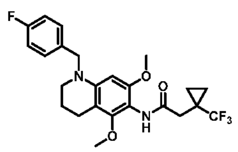 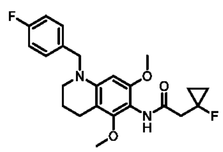 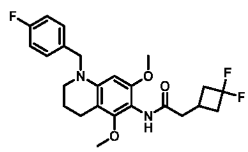 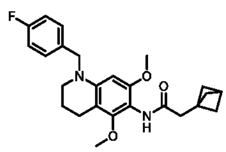

In the second aspect of the present invention, provided is a pharmaceutical composition comprising one or more pharmaceutically acceptable carriers and a safe and effective amount of one or more of the compounds described in the first aspect of the present invention, or a pharmaceutically acceptable salt thereof.

In the third aspect of the present invention, provided is a use of the compound described in the first aspect of the present invention, or a pharmaceutically acceptable salt thereof in the preparation of a drug for the prevention and/or treatment of a disease sensitive to potassium ion channels.

In another preferred embodiment, the disease sensitive to potassium ion channels is a central nervous system disease.

It should be understood that within the scope of the present invention, the above-mentioned technical features of the present invention and the technical features specifically described in the following (such as embodiments) can be combined with each other to form a new or preferred technical solution. Limited to space, it will not be repeated here.

## Detailed Description of the Invention

After long-term and in-depth research, the inventor unexpectedly prepared a compound shown in formula I with excellent potassium channel opening activity, pharmacokinetics (such as brain blood ratio performance), in vivo efficacy and safety, and novel structure through structural optimization. On this basis, the present invention is completed.

## Terms

In the present invention, unless otherwise specified, the terms used herein have the ordinary meanings known to those skilled in the art.

IIn the present invention, the term "halogen" refers to F, Cl, Br or I.

In the present invention, the term "$C_{1-6}$ alkyl" refers to a straight or branched alkyl comprising 1-6 carbon atoms, such as methyl, ethyl, propyl, isopropyl, butyl, isobutyl, tert-butyl, neopentyl, tert-pentyl, and the like.

In the present invention, the term "$C_{2-6}$ alkenyl" refers to a straight or branched alkenyl containing a double bond and 2-6 carbon atoms, and non-limitingly includes vinyl, propenyl, butenyl, isobutenyl, pentenyl, hexenyl, and the like.

In the present invention, the term "$C_{2-6}$ alkynyl" refers to a straight or branched alkynyl containing a triple bond and 2-6 carbon atoms, and non-limitingly includes ethynyl, propynyl, butynyl, isobutynyl, pentynyl, hexynyl and the like.

In the present invention, the term "$C_{3-6}$ cyclic hydrocarbon group" includes groups selected from the group consisting of: $C_{3-6}$ cycloalkyl, $C_{3-6}$ cycloalkenyl, and $C_{3-6}$ cycloalkynyl.

The term "$C_{3-6}$ cycloalkyl" in the present invention refers to a cyclic alkyl having 3-6 carbon atoms on the ring, and non-limitingly includes cyclopropyl, cyclobutyl, cyclopentyl, cyclohexyl, and the like.

In the present invention, the term "$C_{5-8}$ bridged cyclic group" refers to a cycloalkyl having a bridge bond and 5-8 carbon atoms, and non-limitingly includes

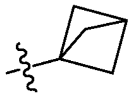,

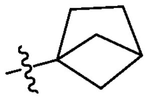, 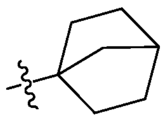,

and the like.

In the present invention, the term "$C_{1-6}$ alkoxy" refers to a straight or branched alkoxy with 1 to 6 carbon atoms, including without limitation methoxy, ethoxy, propoxy, isopropoxy, butoxy, and the like. Preferably refers to $C_{1-4}$ alkoxy.

In the present invention, the term "heterocyclyl" refers to a 4-8-membered heterocyclyl containing 1, 2, or 3 heteroatoms selected from N, O and S, including, but not limited to, the following groups:

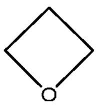, 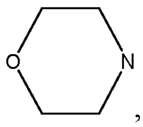, 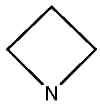, 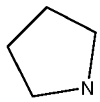 and 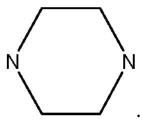.

In the present invention, the term "aromatic ring" or "aryl" has the same meaning, preferably "$C_{6-10}$ aryl". The term "$C_{6-10}$ aryl" refers to an aromatic ring group having 6-10 carbon atoms without heteroatoms on the ring, such as phenyl, naphthyl and the like.

In the present invention, the term "aromatic heterocycle" or "heteroaryl" has the same meaning and refers to a heteroaromatic group containing one to more heteroatoms. For example, "C3-C10 heteroaryl" refers to an aromatic heterocyclic ring containing 1 to 4 heteroatoms selected from oxygen, sulfur, and nitrogen and 3 to 10 carbon atoms. Non-limiting examples include: furanyl, thienyl, pyridinyl, pyrazolyl, pyrrolyl, N-alkylpyrrolyl, pyrimidinyl, pyrazinyl, imidazolyl, tetrazolyl and the like. Said heteroaryl ring may be fused to an aryl, heterocyclyl or cycloalkyl ring, wherein the ring attached to the parent structure is a heteroaryl ring. Heteroaryl can be optionally substituted or unsubstituted.

In the present invention, the term "halogenated" means being substituted by deuterium.

In the present invention, the term "deuterated" means being substituted by deuterium.

In the present invention, the term "substituted" refers to one or more hydrogen atoms on a particular group are substituted by a particular substituent. The particular substituent is a substituent described above, or a substituent appearing in the Examples. Unless specifically stated, a particular substituted group may have a substituent selected from a particular group at any substitutable site of the group, and the substituents may be the same or different at various positions. It should be understood by those skilled in the art that the combinations of substituents contemplated by the present invention are those that are stable or chemically realizable. The substituents are, for example (but not limited to): halogen, hydroxyl, carboxyl (-COOH), $C_1$-$C_6$ alkyl, $C_2$-$C_6$ alkenyl, $C_2$-$C_6$ alkynyl, $C_3$-$C_8$ cycloalkyl, 3- to 12-membered heterocyclyl, aryl, heteroaryl, $C_1$-$C_8$ aldehyde, $C_2$-$C_{10}$ acyl, $C_2$-$C_{10}$ ester group, amino, $C_1$-$C_6$ alkoxy, $C_1$-$C_{10}$ sulfonyl, etc.

In the present invention, the terms 1-6 refer to 1, 2, 3, 4, 5 or 6. Other similar terms have similar meanings independently. The term "more" means 2-6, e.g. 2, 3, 4, 5 or 6.

It should be understood that when a group is present in several different positions of a compound, its definition in each position is independent of each other and may be the same or different. That is, the term "selected from the group

consisting of:" has the same meaning as the term "each independently selected from the group consisting of:".

**Compound**

The present invention provides a compound of formula I, or a pharmaceutically acceptable salt thereof,

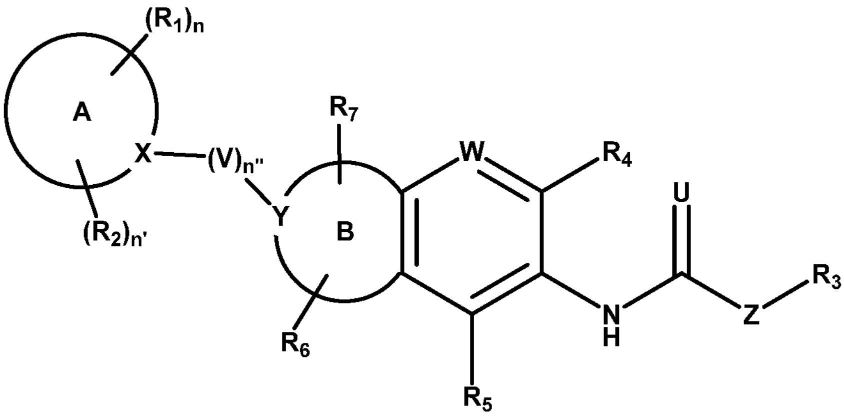

Formula I

wherein, each group is as defined above.

In another preferred embodiment, in the compound, any one of $R_1$, $R_2$, $R_3$, $R_4$, $R_5$, $R_6$, $R_7$, ring A, n, n', X, V, n", Y, ring B, W, U and Z is independently the corresponding functional group in the specific compound.

In another preferred embodiment, the compound is preferably the compound prepared in each example.

As used herein, the term "pharmaceutically acceptable salt" refers to a salt of a compound of the invention formed with an acid or base suitable for use as a drug. Pharmaceutically acceptable salts include inorganic and organic salts. A preferred class of salts are those formed by the compounds of the present invention with acids. Acids suitable for the formation of salts include, but are not limited to: inorganic acids such as hydrochloric, hydrobromic, hydrofluoric, sulphuric, nitric, and phosphoric acids, etc.; organic acids such as formic, acetic, trifluoroacetic, propionic, oxalic, malonic, succinic, fumaric, maleic, lactic, malic, tartaric, citric, picric, benzoic, methanesulphonic, ethanesulfonic, p-toluenesulphonic, benzenesulphonic, naphthalenesulphonic, etc.; and amino acids such as proline, phenylalanine, aspartic acid, glutamic acid, etc..

Another preferred class of salts are salts formed by the compounds of the present invention with a base, such as alkali metal salts (e.g., sodium or potassium salts), alkaline earth metal salts (e.g., magnesium or calcium salts), ammonium salts (e.g., lower alkanolammonium salts, and other pharmaceutically acceptable amine salts), such as methylamine salts, ethylamine salts, propylamine salts, dimethylamine salts, trimethylamine salts, diethylamine salts, triethylamine salts, tert-butylamine salts, ethylenediamine salts, hydroxyethylamine salts, dihydroxyethylamine salts, trihydroxyethylamine salts, and amine salts formed from morpholine, piperazine, and lysine, respectively.

It should be understood that the compound of the present invention can be prepared by the method as described in the following examples, and can also be conveniently made by optionally combining various synthetic methods described in this specification or known in the art, and such combinations can be readily carried out by those skilled in the field to which the present invention belongs.

**Pharmaceutical composition and mode of application**

The pharmaceutical compositions of the present invention comprise a compound of the present invention or a pharmaceutically acceptable salt thereof within a safe and effective amount of range, and a pharmaceutically acceptable excipient or carrier. The "safe and effective amount" refers to the amount of compound that is sufficient to significantly improve the condition without causing serious side effects. Typically, the pharmaceutical composition contains 1-2000mg compound of the present invention /dose, and more preferably, 5-1000mg compound of the present invention/dose. Preferably, the "one dose" is a capsule or tablet.

"Pharmaceutically acceptable carrier" refers to one or more compatible solid or liquid fillers or gel substances, which are suitable for human use and must have sufficient purity and low toxicity. "Compatibility" herein refers to the ability of components of a composition to blend with each other and with the compounds of the invention without significantly reducing the efficacy of the compounds. Some examples of pharmaceutically acceptable carriers include cellulose and its derivatives (such as sodium carboxymethyl cellulose, sodium ethyl cellulose, cellulose acetate, etc.), gelatin, talc, solid lubricants (such as stearic acid, Magnesium stearate), calcium sulfate, vegetable oil (such as soybean oil, sesame oil,

peanut oil, olive oil, etc.), polyols (such as propylene glycol, glycerin, mannitol, sorbitol, etc.), emulsifier (such as Tween®), wetting agents (such as sodium dodecyl sulfate), colorants, flavoring agents, stabilizers, antioxidants, preservatives, non-pyrogen raw water, etc.

The pharmaceutical composition is in the form of injection, wafer, tablet, pill, powders or granules.

The mode of administration of the compounds or pharmaceutical compositions of the present invention are not particularly limited, and representative mode of administration include, but are not limited to, oral, intratumoral, rectal, parenteral (intravenous, intramuscular or subcutaneous), and topical administration.

Solid dosage forms for oral administration include capsules, tablets, pills, powders and granules. In these solid dosage forms, the active compound is mixed with at least one conventional inert excipient (or carrier), such as sodium citrate or dicalcium phosphate, or with the following ingredients: (a) filler or compatibilizer, such as starch, lactose, sucrose, glucose, Mannitol and silicic acid; (b) adhesives, such as hydroxymethyl cellulose, alginate, gelatin, polyvinylpyrrolidone, sucrose and gum arabic; (c) moisturizing agents, such as glycerol; (d) disintegrating agents, such as agar, calcium carbonate, potato starch or cassava starch, alginic acid, some complex silicates, and sodium carbonate; (e) slow solvent, such as paraffin; (f) absorption accelerators, such as quaternary amine compounds; (g) wetting agents, such as cetyl alcohol and glycerol monostearate; (h) adsorbents, such as kaolin; and (i) lubricants, such as talc, calcium stearate, magnesium stearate, solid polyethylene glycol, sodium dodecyl sulfate, or mixtures thereof. In capsules, tablets and pills, dosage forms may also contain buffers.

Solid dosage forms such as tablets, sugar pills, capsules and granules may be prepared using coatings and shell materials such as casings and other materials well known in the art. They may comprise an opacifying agent, and the active compound or compounds in such composition may be released in a delayed manner in a part of the digestive tract. Examples of embedding components that can be employed are polymeric substances and wax substances. If necessary, the active compound may also form a microcapsule with one or more of the excipients described above.

Liquid dosage forms for oral administration include pharmaceutically acceptable emulsions, solutions, suspensions, syrups or tinctures. In addition to the active compounds, the liquid dosage form may contain inert diluents conventionally used in the art, such as water or other solvents, solubilizers and emulsifiers, for example, ethanol, isopropanol, ethyl carbonate, ethyl acetate, propylene glycol, 1,3-butanediol, dimethylformamide and oils, especially cottonseed oil, peanut oil, corn germ oil, olive oil, castor oil and sesame oil, or mixtures thereof.

In addition to these inert diluents, the composition may also contain auxiliaries such as wetting agents, emulsifiers, suspending agents, sweeteners, flavoring agents and spices.

In addition to the active compound, the suspension may comprise suspending agents, such as ethoxylated isooctadecanol, polyoxyethylene sorbitol and dehydrated sorbitol esters, microcrystalline cellulose, methanolic aluminum, agar, and any mixtures thereof.

The composition for parenteral injection may comprise physiologically acceptable sterile aqueous or anhydrous solutions, dispersions, suspensions or emulsions, and sterile powders for redissolution into sterile injectable solutions or dispersions. Suitable aqueous and non-aqueous carriers, diluents, solvents, or excipients include water, ethanol, polyols, and suitable mixtures thereof.

Dosage forms of the compound of the invention for topical administration include ointments, powders, patches, propellants and inhalants. The active ingredient is mixed under sterile conditions with a physiologically acceptable carrier and any preservatives, buffers, or propellants as may be required.

The compound of the present invention can be administered alone or in combination with other pharmaceutically acceptable compounds.

The treatment method of the present invention can be administered alone or in combination with other treatment methods or drugs.

When using the pharmaceutical composition, a safe and effective amount of the compound of the present invention is administered to mammals (such as humans) in need of treatment, wherein the dosage at the time of administration is the pharmaceutically considered effective dose, which is typically 1- 2000 mg per day, more preferably 5-1000mg per day for a 60 kg body weight human. Of course, the specific dosage should also consider the route of administration, the patient's health condition and other factors, which are all within the skill range of skilled doctors.

Compared with the prior art, the main advantages of the present invention include:

(1) The compound exhibits superior pharmacokinetic properties, such as better brain blood ratio, half-life period, exposure level, metabolic stability, etc;
(2) The compound has better potassium ion channel opening activity, better potassium ion channel activation rate, better ion channel selectivity, better in vivo efficacy, and better safety;
(3) The compound is expected to be used in the treatment and/or prevention of diseases and conditions affected by the activity of potassium ion channels.

The present invention is further described below in conjunction with specific embodiments. It is to be understood

that these examples are intended to illustrate the invention only and not to limit the scope of the invention. The experimental methods that do not indicate specific conditions in the following examples usually follow the conventional conditions, such as those described in Sambrook et al., Molecular Cloning: A Laboratory Manual (New York: Cold Spring Harbor Laboratory Press, 1989), or the conditions suggested by the manufacturer. Unless otherwise specified, percentages and parts are calculated by weight.

Unless otherwise defined, all professional and scientific terms used herein have the same meanings as commonly understood by those skilled in the art. In addition, any methods and materials similar or equivalent to those described can be applied to the method of the present invention. The preferred embodiments and materials described herein are for exemplary purposes only.

The experimental materials and reagents used in the following examples are from commercially available sources unless otherwise specified.

**Example 1 Preparation of Compound 3001**

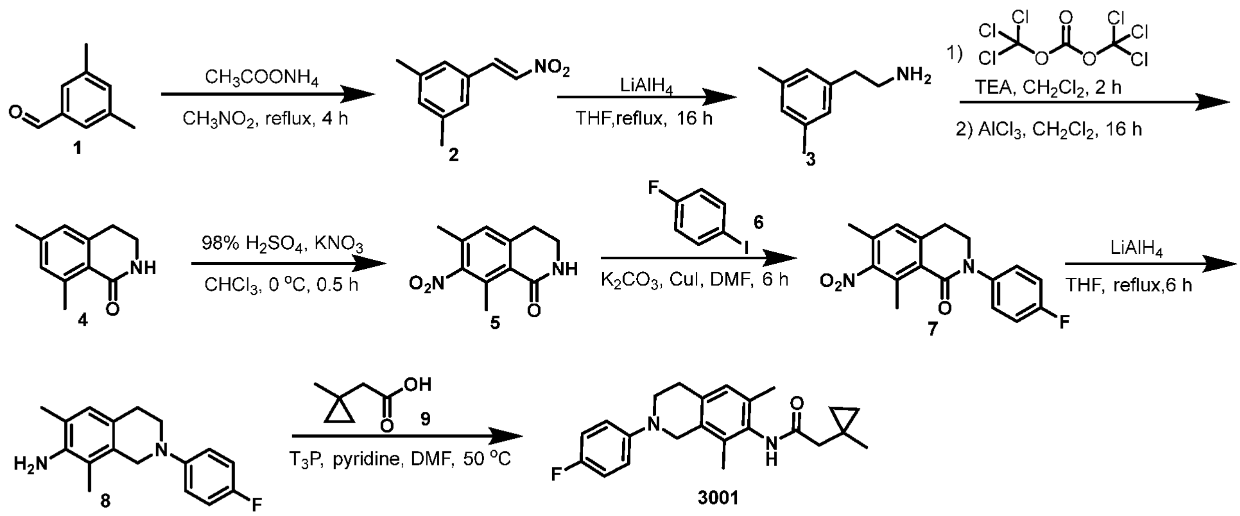


Step 1, compound 2

Compound 1 (5.0 g, 37.3 mmol, 1.0 eq) was dissolved in nitromethane (45 mL) and ammonium acetate (3.4 g, 44.7 mmol, 1.2 eq) was added to the above reaction solution at 25 °C and heated to reflux for 4 hours. After completion of the reaction, the temperature was lowered to 25 °C and the reaction solution was concentrated, and ethyl acetate (80 mL) was added. The organic phase was washed sequentially with water (50 mL), and saturated sodium chloride solution (50 mL), and dried over anhydrous sodium sulfate. The concentrated residue was purified by silica gel column chromatography (petroleum ether/ethyl acetate=100/1) to give compound 2 (3.0 g, 46%) as a yellow solid.
LCMS: $[M+H]^+$ = 178.1

Step 2, compound 3

Compound 2 (2.0 g, 11.3 mmol, 1.0 eq) was dissolved in tetrahydrofuran (30 mL) and the above solution was slowly added dropwise to a solution of lithium tetrahydroaluminate (1 M, 45 mL, 45 mmol, 4.0 eq) in tetrahydrofuran, and after that the reaction solution was heated and refluxed for 16 hours. The reaction solution was then cooled down to 25 °C and quenched with sodium sulfate decahydrate, filtered, and washed with ethyl acetate, and the resulting filtrate was concentrated to give compound 3 (1.6 g, 95%) as a yellow solid.
LCMS: $[M+H]^+$ = 150.1

Step 3, compound 4

Triphosgene (1.3 g, 4.3 mmol, 0.4 eq) was dissolved in dichloromethane (10 mL) and the above solution was slowly added dropwise to a solution of compound 3 (1.6 g, 10.7 mmol, 1.0 eq) in dichloromethane (10 mL), cooled to 0 °C, and triethylamine (2.2 g, 21.5 mmol, 2.0 eq) was added dropwise. The reaction solution was stirred at 25 °C for 2 h and then

filtered. The solids were washed with a small amount of dichloromethane, and the resulting filtrate was added slowly and dropwise to a suspension of aluminum trichloride (5.7 g, 42.9 mmol, 4.0 eq) in dichloromethane (25 mL). Stirring was continued for 16 h and then the reaction was quenched with water; the organic phase was dried over anhydrous sodium sulfate after partitioning, and the residue obtained after concentration was purified by fast silica gel column chromatography (petroleum ether/ethyl acetate = 2/1) to give compound 4 (0.6 g, 32%) as a yellow solid.
LCMS: $[M+H]^+$ = 176.1

Step 4, compound 5

Compound 4 (200 mg, 1.14 mmol, 1.0 eq) was dissolved in chloroform (2 mL) and cooled to 0 °C, and a solution of potassium nitrate (127 mg, 1.26 mmol, 1.1 eq) in concentrated sulfuric acid (98%, 3 mL) was added dropwise. The reaction was stirred at 0 °C for 0.5 h and quenched with crushed ice, and the solid obtained by filtration was purified by pulping with water to give compound 5 (213 mg, 85%) as a yellow solid.
LCMS: $[M+H]^+$ = 221.1

Step 5, compound 7

Compound 5 (280 mg, 1.27 mmol, 1.0 eq) was dissolved in N,N-dimethylformamide (5 mL) and compound 6 (339 mg, 1.53 mmol, 1.2 eq), cuprous iodide (48 mg, 0.25 mmol, 0.2 eq), and potassium carbonate (352 mg, 2.54 mmol, 2.0 eq) were added sequentially. The reaction solution was heated to 140 °C under nitrogen atmosphere and stirred for 6 h. The reaction solution was cooled to 25 °C and diluted with ethyl acetate (10 mL). The reaction solution was filtered, the solid was washed with ethyl acetate, the resulting filtrate was washed with water (10 mL), the organic phase was dried over anhydrous sodium sulfate and concentrated, and the residue was purified by fast silica gel column chromatography (petroleum ether/ethyl acetate=10/1) to give compound 7 (145 mg, 36%) as a yellow solid.
LCMS: $[M+H]^+$ = 315.1

Step 6, compound 8

Compound 7 (530 mg, 1.69 mmol, 1.0 eq) was dissolved in tetrahydrofuran (70 mL), and the above solution was slowly added dropwise to a solution of lithium tetrahydroaluminate (1M, 17 mL, 17 mmol, 10.0 eq) in tetrahydrofuran. After addition, the reaction was refluxed at elevated temperature for 6 hours. After cooling to 25 °C, the reaction was quenched with sodium sulfate decahydrate, the solid was washed with ethyl acetate after filtration and the resulting filtrate was concentrated to give compound 8 (423 mg, 93%) as a yellow solid.
LCMS: $[M+H]^+$ = 271.1

Step 7, compound 3001

Compound 8 (200 mg, 0.74 mmol, 1.0 eq) was dissolved in N,N-dimethylformamide (6 mL), and compound 9 (102 mg, 0.89 mmol, 1.2 eq), pyridine (1170 mg, 14.8 mmol, 20.0 eq) and 1-propylphosphonic anhydride (50% ethyl acetate solution, 4.8 g, 7.4 mmol, 10.0 eq) were added sequentially, then heated to 50 °C and stirred for 16 hours. After cooling to 25 °C, the reaction solution was diluted with ethyl acetate; the organic phase was washed sequentially with water and saturated sodium chloride solution, and dried with anhydrous sodium sulfate; and the residue obtained after concentration was purified by silica gel column chromatography (petroleum ether/ethyl acetate=2/1) to give compound **3001** (139.1 mg, 52%) as a light yellow solid.
LCMS: $[M+H]^+$ = 367.1 $^1$H NMR (400 MHz, DMSO-d6) δ 9.03 (s, 1H), 7.08-7.06 (m, 4H), 6.88 (s, 1H), 4.16 (s, 2H), 3.42 (t, J = 5.6 Hz, 2H), 2.86 (t, J = 5.6 Hz, 2H), 2.22 (s, 2H), 2.11 (s, 3H), 2.07 (s, 3H), 1.16 (s, 3H), 0.56-0.54 (m, 2H), 0.34-0.31 (m, 2H).

**Example 2 Preparation of Compound 3002**

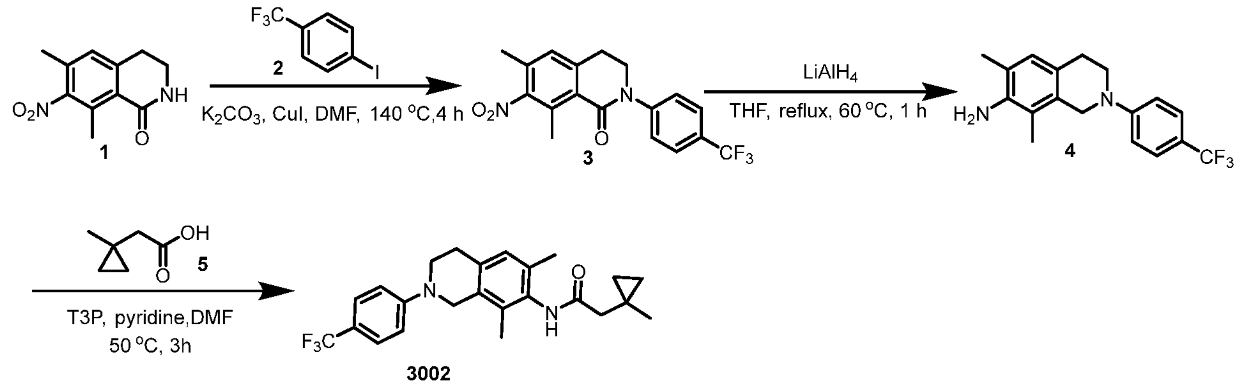


Step 1, compound 3

Compound **1** (500 mg, 2.27 mmol, 1.0 eq) was dissolved in N,N-dimethylformamide (5 mL), and compound **2** (926 mg, 3.40 mmol, 1.5 eq), potassium carbonate (784 mg, 5.68 mmol, 2.5 eq) and cuprous iodide (86 mg, 0.45 mmol, 0.2 eq) were added sequentially, and then the reaction solution was heated to 140 °C and stirred for 4 hours. The reaction solution was cooled to 25 °C, then added with water (50 mL), and extracted with ethyl acetate (3 × 50 mL); the combined organic phases were washed with saturated sodium chloride solution, dried over anhydrous sodium sulfate and then concentrated, and the residue was purified by silica gel column chromatography (petroleum ether/ethyl acetate=10/1) to give compound 3 (400 mg, 48%) as a white solid. LCMS: $[M+H]^+$ =365.1

Step 2, compound 4

Compound **3** (200 mg, 0.548 mmol, 1.0 eq) was dissolved in tetrahydrofuran (2 mL) and cooled to 0 °C, and a solution of lithium tetrahydroaluminate (1 M, 5.48 mL, 5.48 mmol, 10.0 eq) in tetrahydrofuran was added dropwise. After addition, the reaction solution was warmed up to 60 °C and stirred for 1 hour. The reaction solution was cooled to 0 °C and then quenched with sodium sulfate decahydrate. The filtrate obtained after filtration was concentrated to give compound **4** (150 mg, 85%) as a white solid.
LCMS: $[M+H]^+$ =321.1

Step 3, compound 3002

Compound **4** (150 mg, 0.468 mmol, 1.0 eq) was dissolved in N,N-dimethylformamide (5 mL), and compound **5** (64 mg, 0.561 mmol, 1.2 eq), 1-propylphosphonic anhydride (50%, 3.5 g, 5.500 mmol, 11.7 eq) and pyridine (740 mg, 9.36 mmol, 20.0 eq) were added sequentially. The reaction solution was heated to 50 °C and stirred for 2 h, then cooled to 25 °C, added with water (50 mL) and extracted with ethyl acetate (3 × 50 mL). The combined organic phases were washed with saturated sodium chloride solution, dried over anhydrous sodium sulfate and concentrated, and the residue was purified by pre-HPLC (0.1% formic acid/acetonitrile/water) to give compound **3002** (40 mg, 20%) as a white solid.

LCMS: $[M+H]^+$ =417.3
$^1H$ NMR (400 MHz, DMSO-$d_6$) δ 9.05 (s, 1H), 7.52 (d, *J* = 8.8 Hz, 2H), 7.16 (d, *J* = 8.8 Hz, 2H), 6.91 (s, 1H), 4.35 (s, 2H), 3.60 (t, *J* = 5.5 Hz, 2H), 2.87 (t, *J* = 5.6 Hz, 2H), 2.23 (s, 2H), 2.11 (d, *J* = 4.6 Hz, 6H), 1.16 (s, 3H), 0.56-0.54 (m, 2H), 0.34-0.31 (m, 2H).

**Example A1 Potassium Ion Channel Opener Activity Test (FDSS/μ CELL Detection)**

1. Experimental Method:

1.1 Experimental Procedure

Cell preparation: CHO-KCNQ2 cells were cultured in a 175 $cm^2$ culture flask, and when the cell density grew to 60-80%, the culture medium was removed and the cells were washed once with 7 mL PBS (Phosphate Buffered Saline), and then 3 mL of 0.25% Trypsin was added for digestion. After completion of digestion, 7 mL of culture medium (90% DMEM/F12 + 10% FBS + 500 μg/mL G418) was added to neutralize, and centrifuged at 800 rpm for 3 min; the supernatant was aspirated, then 5 mL of culture medium was added to resuspend the cells, and the cells were counted.

Cell spreading: according to the cell counting results, the density was adjusted to $3\times10^4$ cells/well. After standing at room temperature for 30 minutes, the cells were placed in a COz incubator at 37°C overnight, and incubated for 16-18 hours to reach a cell density of about 80%.
Fluorescent dye incubation: Cell culture solution was discarded, 80 μL/well of loading buffer was added and the cells were incubated for 60 minutes at room temperature and protected from light.
Compound incubation: Loading buffer was discarded, 80 μL/well of prepared compound solution was added, and the cells were incubated for 20 min at room temperature and protected from light.
Fluorescence data acquisition: Real-time fluorescence signal recording was performed using an FDSS/μCELL instrument with an excitation wavelength of 480 nm and an emission wavelength of 540 nm, and the signal was recorded once per second. 10 seconds of the baseline was recorded, then 20 μL/well of stimulation buffer was added, and the recording was continued until the end of 180 seconds.

1.2 Solution Preparation

Loading buffer: 10mL/plate, prepared as follows:

| Component | Volume |
|---|---|
| PowerLoad™ concentrate, 100X (component C) | 100 μL |
| FluxOR™ reagent, reconstructed in DMSO (step 1.2) | 10 μL |
| Deionized water | 8.8 mL |
| FluxOR™ test buffer, 10X (component B) | 1 mL |
| probenecid, reconstructed in deionized water (step 1.1) | 100 μL |
| Total Volume | 10mL |

Test buffer: 100mL/plate, prepared as follows:

| Component | Volume |
|---|---|
| Deionized water | 8.9 mL |
| FluxOR™ test buffer, 10X (component B) | 1 mL |
| probenecid, reconstructed in deionized water (step 1.1) | 100 μL |
| Total Volume | 10 mL |

Stimulation buffer: 5 mL/plate, prepared as follows:

| Component | Volume | |
|---|---|---|
| | $+ K^+$ | $- K^+$ |
| Deionized water | 2.5 mL | 3.5 mL |
| FluxOR™ Chlorine Free Buffer, 5X (Component E) | 1 mL | 1 mL |
| $K_2SO_4$ Concentrate (125 mM concentrated solution of $K_2SO_4$, Component F) | 1 mL | / |
| $Tl_2SO_4$ concentrate (50 mM concentrated solution of $Tl_2SO_4$, component G) | 0.5 mL | 0.5 mL |
| Total Volume | 5 mL | 5 mL |

The above buffers were obtained from a commercially available kit called FluxOR potassium ion channel assay.

1.3 Compound preparation

20 mM stock solution of compound in DMSO was prepared, 10 μL of 20 mM compound stock solution was taken into 20 μL of DMSO solution, and a 3-fold serial dilution was performed to form 8 intermediate concentrations; then the

intermediate concentrations of the compounds were taken respectively into test buffer, and a 200-fold dilution was performed to obtain the final concentration to be tested, and 80 μL of compound was taken and added to the assay plate.

The highest test concentration was 100 μM, and there was a total of8 concentrations, i.e., 100, 33.33, 11.11, 3.70, 1.23, 0.41, 0.137, 0.045μM, respectively, with 3 replicate wells for each concentration.

The amount of DMSO in the final test concentration did not exceed 0.5%, and this concentration of DMSO had no effect on KCNQ2 potassium channels.

1.4 Data analysis

The experimental data were analyzed by Excel 2007, GraphPad Prism 5.0 software, and the ratio of 180 s was counted to calculate the agonistic effect. The compound agonistic effect was calculated by the following equation:

$$\text{Percent of agonism} = \frac{\text{Ratio of fluorescence signal with compound - Ratio of fluorescence signal without compound}}{\text{Ratio of fluorescence signal without compound}} \times 100\%$$

1.5 Quality Control

Environment: Temperature ~25 °C
Reagent: FluxORTM detection kit (Invitrogen, Cat # F0017)

The experimental data in the report must meet the following criteria: Z' Factor > 0.5

2. Results: See Table 1 for details, wherein a smaller $EC_{50}$ indicates a higher activity of the corresponding compound.

Table 1. Test results of some of the compounds of the present invention

| Compound | Maximum agonist rate (%) | $EC_{50}$(uM) |
|---|---|---|
| 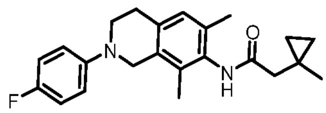 **3001** | 36.30 | 0.40 |
| 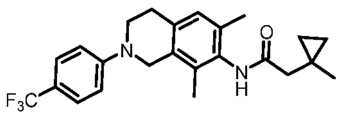 **3002** | 38.96 | 0.059 |

Reference for the above test method:

Zhaobing Gao et al, Journal of Biological Chemistry. 2010, 285(36): 28322-28332.

All documents referred to in the present invention are incorporated by reference herein as if each document is individually incorporated by reference. Further, it should be understood that upon reading the above teachings of the present invention, various modifications or changes may be made to the present invention by those skilled in the art, and those equivalents also fall within the scope defined by the appended claims of the present application.

## Claims

**1.** A compound of formula I, or a pharmaceutically acceptable salt thereof,

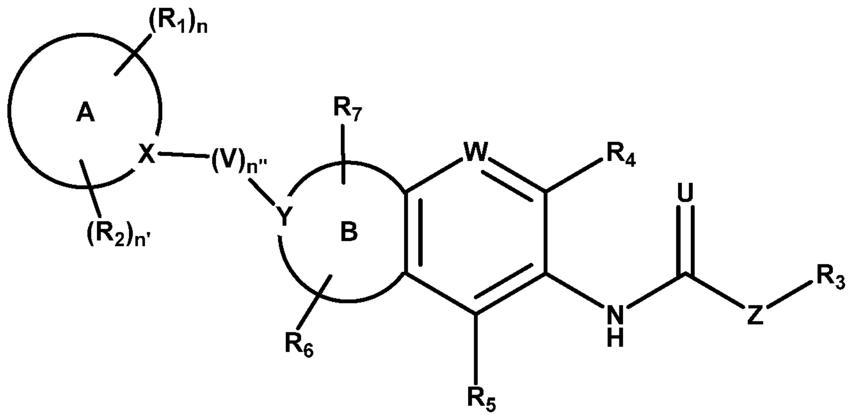

Formula I

wherein,

Ring A is selected from the group consisting of: $C_{6-10}$ aryl, 4-7-membered heteroaryl containing 1-3 heteroatoms selected from N, O and S, saturated or unsaturated $C_{3-6}$ cyclic hydrocarbon group, and 3-10-membered heterocyclyl containing 1-3 heteroatoms selected from N, O and S;

each $R_1$, and $R_2$ are independently selected from the substituted or unsubstituted group consisting of: hydrogen, deuterium, halogen, cyano, -OH, -COOH, nitro, $C_{1-6}$ alkyl, $C_{3-6}$ cycloalkyl, $C_{1-6}$ alkoxy, $C_{3-6}$ cycloalkyloxy, $C_{2-6}$ alkenyl, $C_{2-6}$ alkynyl, saturated or unsaturated $C_{3-6}$ cyclic hydrocarbon group, 3-10-membered heterocyclyl containing 1-3 heteroatoms selected from N, O and S, $C_{6-10}$ aryl, 5-14-membered heteroaryl containing 1-3 heteroatoms selected from N, O and S, $C_{6-12}$ arylalkyl, -N($R_1$')($R_2$'), -C(O)-$R_1$', -C(O)-N($R_1$')($R_2$'), -C(O)-O$R_1$', -N($R_1$')-C(O)-$R_2$', -S(O)$_m$-$R_1$', -S(O)$_m$-N($R_1$')($R_2$'), -S(O)$_m$-O$R_1$', -N($R_1$')-S(O)$_m$- $R_2$', and the "substituted" refers to being substituted by one or more substituents selected from the group consisting of: halogen, $C_{1-6}$ alkyl, $C_{3-6}$ cycloalkyl, $C_{1-6}$ alkoxy, $C_{3-6}$ cycloalkyloxy, $C_{1-6}$ haloalkyl, $C_{3-6}$ halocycloalkyl, $C_{1-6}$ haloalkoxy and $C_{3-6}$ halo-cycloalkyloxy;

n is selected from the group consisting of: 0, 1 and 2;

n' is selected from the group consisting of: 0, 1 and 2;

each $R_1$' and $R_2$' are independently selected from the group consisting of: hydrogen, $C_{1-6}$ alkyl, $C_{3-6}$ cycloalkyl, or $R_1$' and $R_2$' together with the attached N-atom form a saturated or unsaturated 3-10-membered heterocyclyl containing 1-3 heteroatoms selected from N, O and S; and the above alkyl, cycloalkyl and heterocyclyl are optionally substituted by one or more substituents selected from the group consisting of: =O, halogen, $C_{1-6}$ alkyl and $C_{3-6}$ cycloalkyl;

m is selected from the group consisting of: 1 and 2;

X is selected from the group consisting of: C, CRs and N;

each $R_8$ is independently selected from the group consisting of: H, $C_{1-6}$ alkyl, $C_{3-6}$ cycloalkyl, $C_{2-6}$ alkenyl and $C_{2-6}$ alkynyl; and the above alkyl and cycloalkyl are optionally substituted by one or more substituents selected from the group consisting of: halogen, $C_{1-6}$ alkyl and $C_{3-6}$ cycloalkyl;

V is selected from the group consisting of: -C($R_9$)($R_{10}$) - and -N(Rs)-;

n" is selected from the group consisting of: 0 and 1;

$R_9$ and $R_{10}$ are independently selected from the group consisting of: hydrogen, halogen and $C_{1-6}$ alkyl, or $R_9$ and $R_{10}$ together with the attached C-atom form a $C_{3-6}$ cycloalkyl;

ring B is selected from the group consisting of: saturated or unsaturated $C_{3-10}$ cyclic hydrocarbon group, and saturated or unsaturated 3-10 membered heterocyclyl containing 1-3 heteroatoms selected from N, O and S;

$R_6$ and $R_7$ are independently selected from the group consisting of: hydrogen, deuterium, halogen, $C_{1-6}$ alkyl, $C_{3-6}$ cycloalkyl, $C_{2-6}$ alkenyl, $C_{2-6}$ alkynyl, $C_{1-6}$ haloalkyl and $C_{3-6}$ halocycloalkyl;

Y is selected from the group consisting of: CRs and N;

W is selected from the group consisting of: $CR_{11}$ and N;

$R_{11}$ is selected from the group consisting of: hydrogen, deuterium, halogen, cyano, amino, $C_{1-6}$ alkyl, $C_{3-6}$ cycloalkyl, $C_{1-6}$ alkoxy, $C_{3-6}$ cycloalkyloxy, -N($R_1$')($R_2$'); and the above alkyl, cycloalkyl and alkoxy are optionally substituted by one or more substituents selected from the group consisting of: halogen, $C_{1-6}$ alkyl and $C_{3-6}$ cycloalkyl;

$R_4$ and $R_5$ are independently selected from the group consisting of: hydrogen, deuterium, halogen, cyano, amino, $C_{1-6}$ alkyl, $C_{3-6}$ cycloalkyl, $C_{1-6}$ alkoxy, $C_{3-6}$ cycloalkyloxy and -N($R_1$')($R_2$'); and the above alkyl, cycloalkyl and alkoxy are optionally substituted by one or more substituents selected from the group consisting of: halogen, $C_{1-6}$

alkyl and $C_{3-6}$ cycloalkyl;
U is selected from the group consisting of: O, S and N ($R_1$');
Z is selected from the group consisting of: O, $-(CH_2)_q-$ and $-N(R_1')-$;
q is selected from the group consisting of: 0, 1 and 2;
$R_3$ is selected from the group consisting of: $C_{1-6}$alkyl, $C_{3-6}$cycloalkyl, $C_{5-8}$ bridged cyclic group, adamantyl, $C_{6-10}$ aryl, 3-10-membered heteroaryl containing 1-3 heteroatoms selected from N, O and S, 4-8-membered heterocycloalkyl containing 1-3 heteroatoms selected from N, O and S, $C_{3-6}$ cycloalkenyl, $C_{2-6}$ alkenyl and $C_{2-6}$ alkynyl; and the above alkyl, cycloalkyl, bridged cyclic group, adamantyl, aryl, heteroaryl, heterocycloalkyl, cycloalkenyl, alkenyl, and alkynyl are optionally substituted by one or more substituents selected from the group consisting of: hydrogen, halogen, cyano, nitro, amino, hydroxyl, $C_{1-6}$ alkyl-CO-, $C_{1-6}$ alkyl, $C_{3-6}$ cycloalkyl, $C_{6-10}$ aryl, $C_{1-6}$ haloalkyl, $C_{1-6}$ alkoxy, $C_{1-6}$ alkylamino and $C_{1-6}$ haloalkoxy.

**2.** The compound according to claim 1, or a pharmaceutically acceptable salt thereof, wherein,

ring A is selected from the group consisting of: $C_{6-10}$ aryl and 4-7-membered heteroaryl containing 1-3 heteroatoms selected from N, O and S;
each $R_1$, and $R_2$ are independently selected from the substituted or unsubstituted group consisting of: hydrogen, deuterium, halogen, cyano, $C_{1-6}$ alkyl, $C_{3-6}$ cycloalkyl, $C_{1-6}$ alkoxy, $C_{3-6}$ cycloalkyloxy, $C_{2-6}$ alkenyl, $C_{2-6}$ alkynyl and $-N(R_1')(R_2')$, and the "substituted"refers to being substituted by one or more substituents selected from halogen;
n is selected from the group consisting of: 0, 1 and 2;
n' is selected from the group consisting of: 0, 1 and 2;
each $R_1$' and $R_2$' are independently selected from the group consisting of: hydrogen, $C_{1-6}$ alkyl and $C_{3-6}$ cycloalkyl; the above alkyl and cycloalkyl are optionally substituted by one or more substituents selected from halogen;
X is C;
V is $CH_2$;
n" is selected from the group consisting of: 0 and 1;
ring B is a saturated or unsaturated 3-10-membered heterocyclyl containing 1-3 heteroatoms selected from N, O and S;
$R_6$ and $R_7$ are independently selected from the group consisting of: hydrogen and deuterium;
YisN;
W is selected from the group consisting of: $CR_{11}$ and N;
$R_{11}$ is selected from the group consisting of: hydrogen, halogen and $C_{1-6}$alkyl; and the above alkyl is optionally substituted by one or more substituents selected from halogen;
$R_4$ and $R_5$ are independently selected from the group consisting of: hydrogen, halogen, cyano, amino, $C_{1-6}$ alkyl, $C_{3-6}$ cycloalkyl, $C_{1-6}$ alkoxy and $C_{3-6}$ cycloalkyloxy; and the above alkyl, cycloalkyl and alkoxy are optionally substituted by one or more substituents selected from halogen;
U is O;
Z is selected from the group consisting of: O and $CH_2$;
$R_3$ is selected from the group consisting of: $C_{1-6}$ alkyl, $C_{3-6}$ cycloalkyl, $C_{5-8}$ bridged cyclic group and $C_{2-6}$ alkynyl; and the above alkyl, cycloalkyl, bridged cyclic group, and alkynyl are optionally substituted by one or more substituents selected from the group consisting of: hydrogen, halogen, $C_{1-6}$ alkyl, $C_{3-6}$ cycloalkyl, $C_{1-6}$ haloalkyl, $C_{1-6}$ alkoxy and $C_{1-6}$ haloalkoxy.

**3.** The compound according to claim 2, or a pharmaceutically acceptable salt thereof, wherein,

ring A is $C_{6-10}$ aryl;
each $R_1$ and $R_2$ are independently selected from the substituted or unsubstituted group consisting of: hydrogen, halogen, $C_{1-6}$ alkyl, $C_{3-6}$ cycloalkyl, $C_{1-6}$ alkoxy, $C_{3-6}$ cycloalkyloxy, $C_{2-6}$ alkynyl and $-N(R_1')(R_2')$, and the "substituted"refers to being substituted by one or more substituents selected from halogen;
n is selected from the group consisting of: 0, 1 and 2;
n' is selected from the group consisting of: 0, 1 and 2;
each $R_1$' and $R_2$' are independently selected from the group consisting of: hydrogen and $C_{1-6}$ alkyl;
X is C;
V is $CH_2$;
n" is selected from the group consisting of: 0 and 1;
ring B is a saturated or unsaturated 3-10-membered heterocyclyl containing 1-3 heteroatoms selected from N, O

and S;
$R_6$ and $R_7$ are independently selected from the group consisting of: hydrogen and deuterium;
YisN;
W is CH;
$R_4$ and $R_5$ are independently selected from the group consisting of: halogen, $C_{1-6}$alkyl and $C_{1-6}$alkoxy; and the above alkyl and alkoxy are optionally substituted by one or more substituents selected from halogen;
U is O;
Z is $CH_2$;
$R_3$ is selected from the group consisting of: $C_{3-6}$ cycloalkyl and $C_{5-8}$ bridged cyclic group; and the above cycloalkyl and bridged cyclic group are optionally substituted by one or more substituents selected from the group consisting of: hydrogen, halogen, $C_{1-6}$alkyl and $C_{1-6}$haloalkyl.

**4.** The compound according to claim 3, or a pharmaceutically acceptable salt thereof, wherein,

ring A is phenyl;
each $R_1$ and $R_2$ are independently selected from the substituted or unsubstituted group consisting of: hydrogen, halogen, $C_{1-6}$ alkyl, $C_{1-6}$ alkoxy, $C_{2-6}$ alkynyl, -N($R_1$')($R_2$'), and the "substituted" refers to being substituted by one or more substituents selected from halogen;
n is selected from the group consisting of: 0, 1 and 2;
n' is selected from the group consisting of: 0, 1 and 2;
each $R_1$' and $R_2$' are independently selected from the group consisting of: hydrogen and $C_{1-6}$ alkyl;
X is C;
n" is 0;
ring B is selected from the group consisting of:

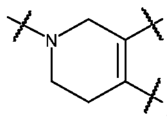, 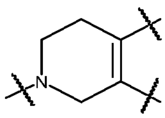, 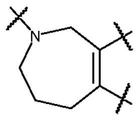,

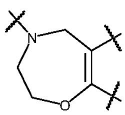, 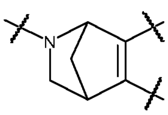, 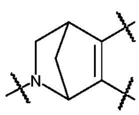, 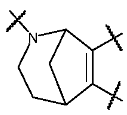, 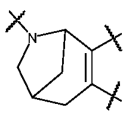, 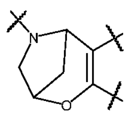, 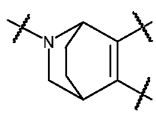,

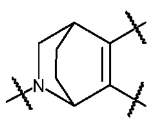, 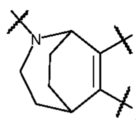, 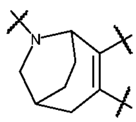 and 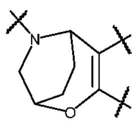;

$R_6$ and $R_7$ are independently selected from the group consisting of: hydrogen and deuterium;
W is CH;
$R_4$ and $R_5$ are independently selected from the group consisting of: halogen, $C_{1-6}$alkyl and $C_{1-6}$alkoxy; and the above alkyl and alkoxy are optionally substituted by one or more substituents selected from halogen;
U is O;
Z is $-CH_2-$;
$R_3$ is selected from the group consisting of: $C_{3-6}$ cycloalkyl and $C_{5-8}$ bridged cyclic group; and the above cycloalkyl and bridged cyclic group are optionally substituted by one or more substituents selected from the group consisting of: hydrogen, halogen, $C_{1-6}$alkyl and $C_{1-6}$haloalkyl.

**5.** The compound according to claim 4, or a pharmaceutically acceptable salt thereof, wherein,

ring A is phenyl;
each $R_1$ and $R_2$ are independently selected from the substituted or unsubstituted group consisting of: hydrogen, halogen, $C_{1-6}$ alkyl, $C_{1-6}$ alkoxy, $C_{2-6}$ alkynyl, and -N($R_1$')($R_2$'), and the "substituted" refers to being substituted by one or more substituents selected from halogen;
n is selected from the group consisting of: 0, 1 and 2;

n' is selected from the group consisting of: 0, 1 and 2;
each $R_1$' and $R_2$' are independently selected from the group consisting of: hydrogen and $C_{1-6}$ alkyl;
X is C;
n" is 0;
ring B is selected from the group consisting of:

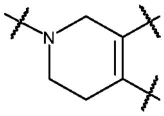,
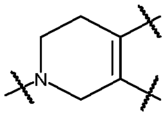

and

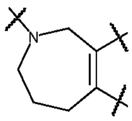;

$R_6$ and $R_7$ are independently selected from the group consisting of: hydrogen and deuterium;
W is CH;
$R_4$ and $R_5$ are independently selected from the group consisting of: halogen, $C_{1-6}$alkyl and $C_{1-6}$alkoxy; and the above alkyl and alkoxy are optionally substituted by one or more substituents selected from halogen;
U is O;
Z is $-CH_2-$;
$R_3$ is selected from the group consisting of: $C_{3-6}$ cycloalkyl and $C_{5-8}$ bridged cyclic group; and the above cycloalkyl and bridged cyclic group are optionally substituted by one or more substituents selected from the group consisting of: hydrogen, halogen, $C_{1-6}$alkyl and $C_{1-6}$haloalkyl.

**6.** The compound according to claim 3, or a pharmaceutically acceptable salt thereof, wherein,

ring A is phenyl;
each $R_1$ and $R_2$ are independently selected from the substituted or unsubstituted group consisting of: hydrogen, halogen, $C_{1-6}$ alkyl, $C_{1-6}$ alkoxy, $C_{2-6}$ alkynyl, and $-N(R_1')(R_2')$, and the "substituted" refers to being substituted by one or more substituents selected from halogen;
n is selected from the group consisting of: 0, 1 and 2;
n' is selected from the group consisting of: 0, 1 and 2;
each $R_1$' and $R_2$' are independently selected from the group consisting of: hydrogen and $C_{1-6}$ alkyl;
X is C;
V is $-CH_2-$;
n" is 1
ring B is selected from the group consisting of:

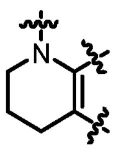,
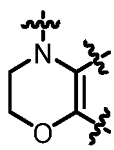,
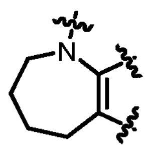

and

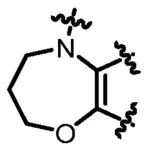;

$R_6$ and $R_7$ are independently selected from the group consisting of: hydrogen and deuterium;
W is CH;

$R_4$ and $R_5$ are independently selected from the group consisting of: halogen, $C_{1-6}$alkyl and $C_{1-6}$alkoxy; and the above alkyl and alkoxy are optionally substituted by one or more substituents selected from halogen;
U is O;
Z is $-CH_2-$;
$R_3$ is selected from the group consisting of: $C_{3-6}$ cycloalkyl and $C_{5-8}$ bridged cyclic group; and the above cycloalkyl and bridged cyclic group are optionally substituted by one or more substituents selected from the group consisting of: hydrogen, halogen, $C_{1-6}$alkyl and $C_{1-6}$haloalkyl.

**7.** The compound according to claim 1, or a pharmaceutically acceptable salt thereof, wherein, the compound is selected from the group consisting of:

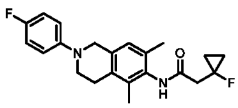
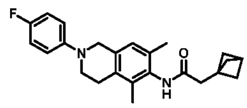
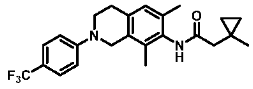

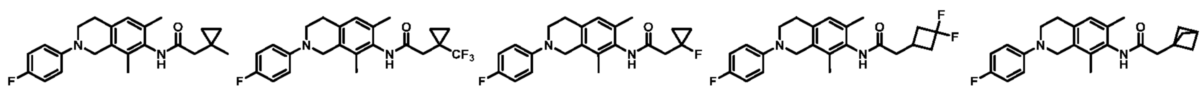

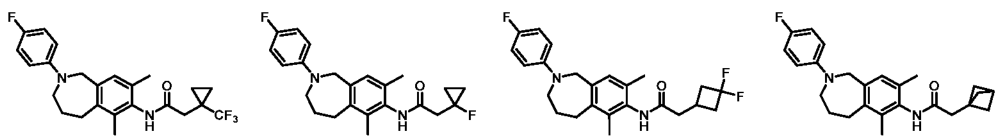

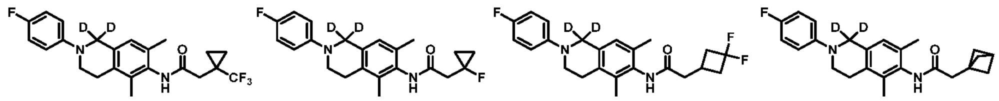

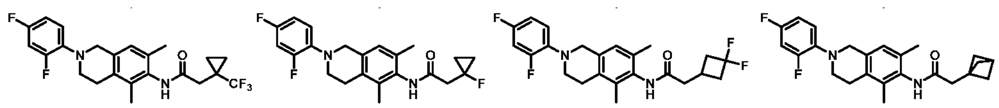

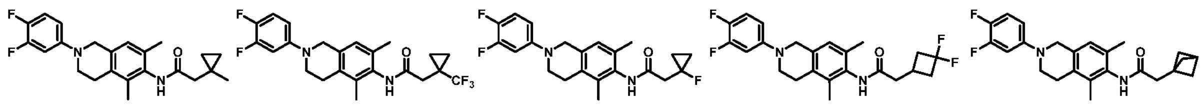

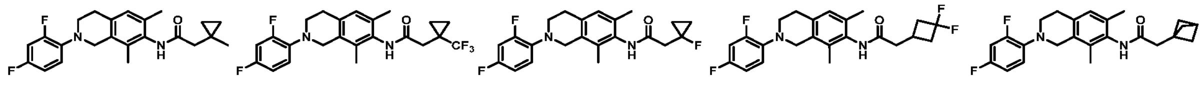

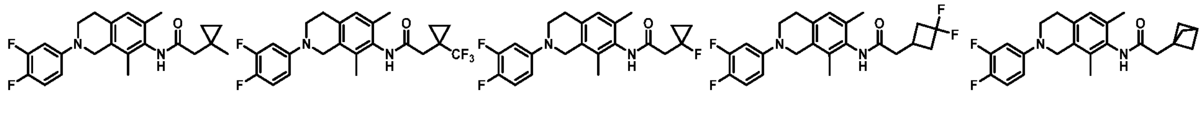

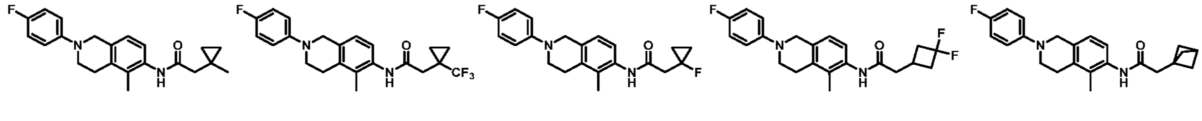

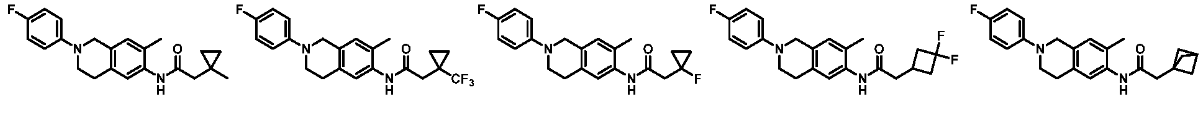

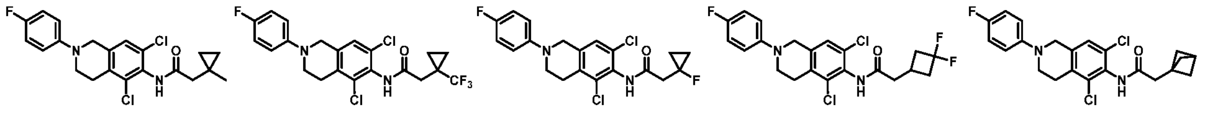

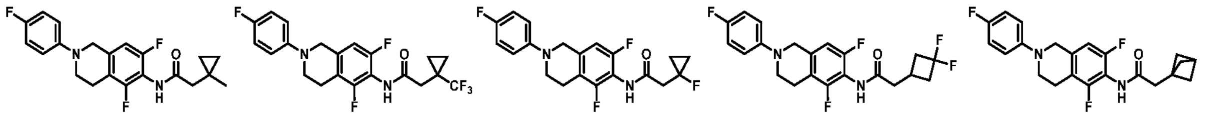

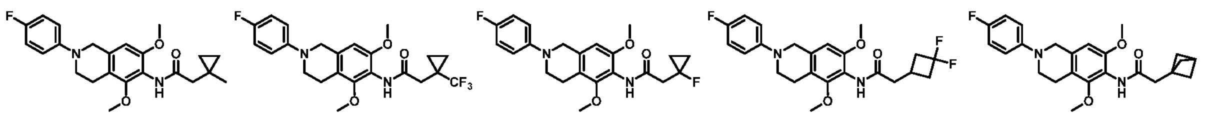

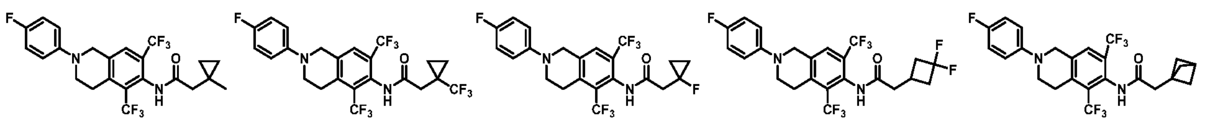

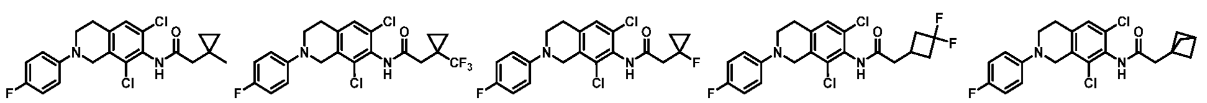

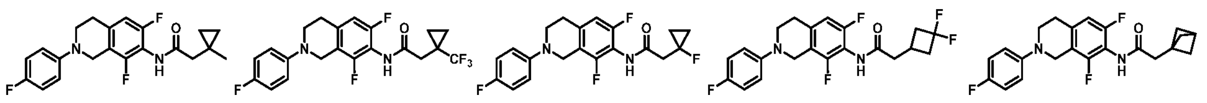

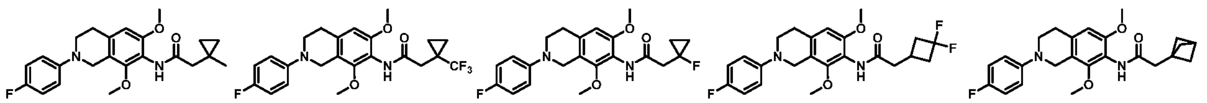

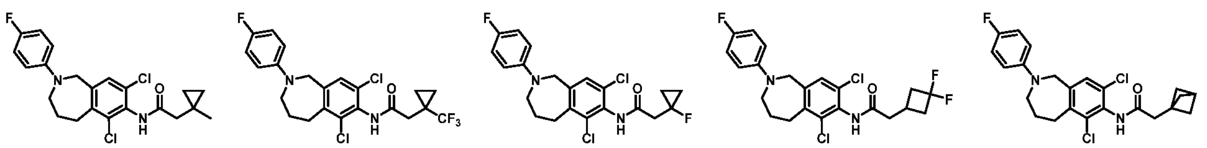

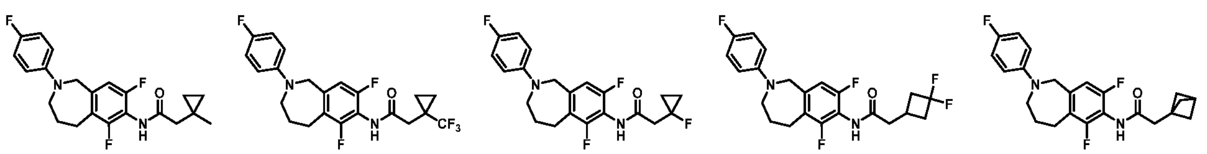

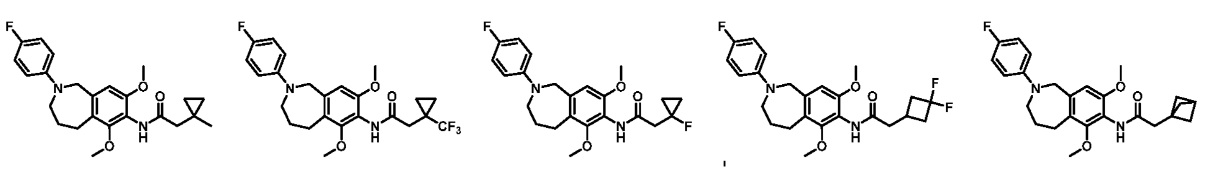

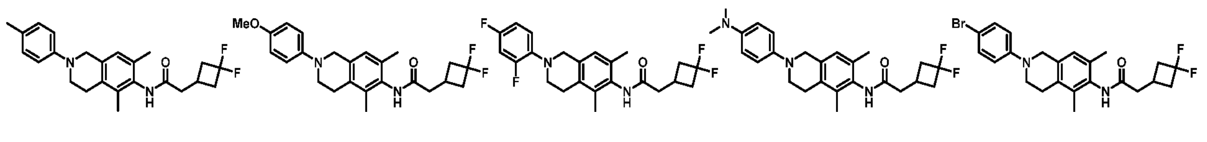

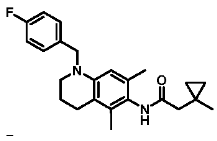 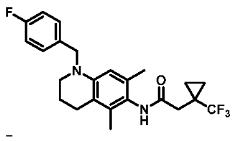 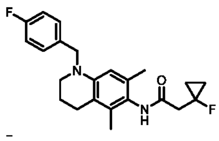 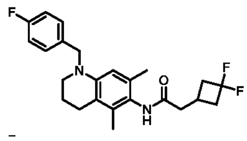 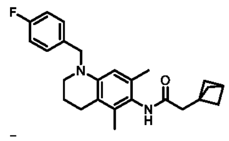

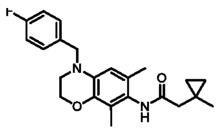 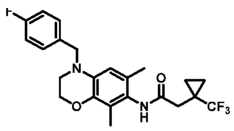 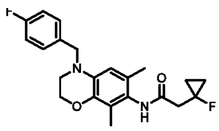 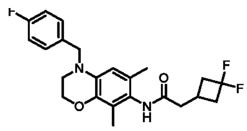 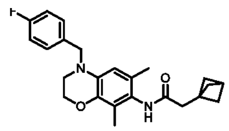

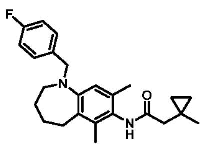 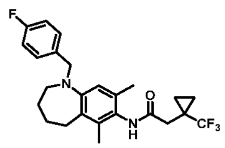 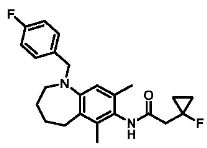 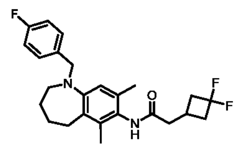 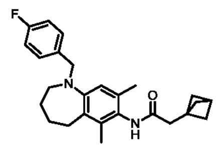

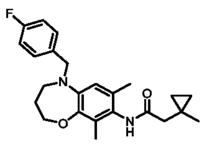 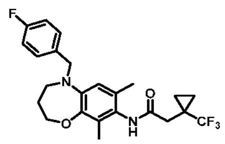 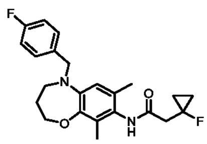 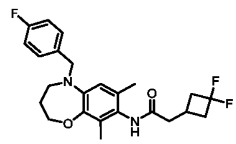 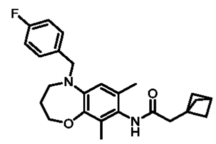

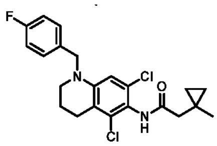 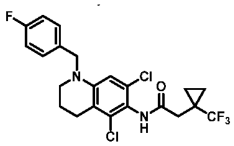 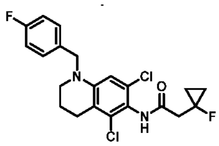 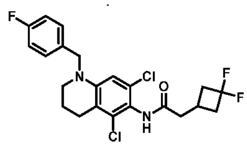 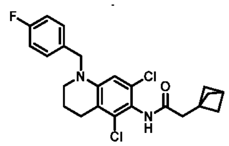

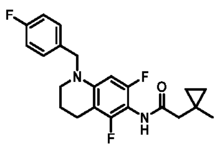 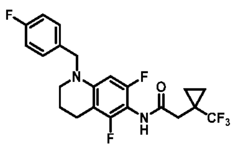 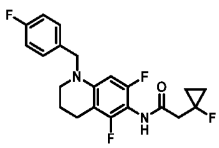 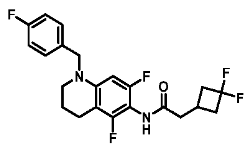 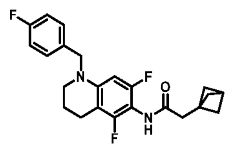

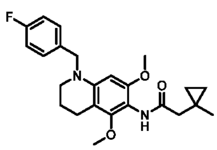 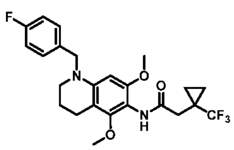 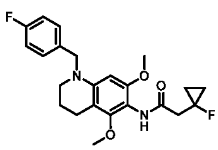 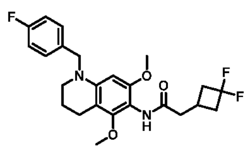 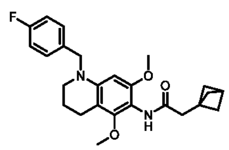

**8.** A pharmaceutical composition comprising one or more pharmaceutically acceptable carriers and a safe and effective amount of one or more of the compounds according to claim 1 or a pharmaceutically acceptable salt thereof.

**9.** A use of the compound according to claim 1, or a pharmaceutically acceptable salt thereof in the preparation of a drug for the prevention and/or treatment of a disease sensitive to potassium ion channels.

**10.** The use according to claim 9, wherein the disease sensitive to potassium ion channels is a central nervous system disease.

## INTERNATIONAL SEARCH REPORT

International application No.
**PCT/CN2023/073185**

**A. CLASSIFICATION OF SUBJECT MATTER**

C07D217/04(2006.01)i;C07D223/16(2006.01)i;C07D215/06(2006.01)i;C07D265/36(2006.01)i;C07D267/14(2006.01)i; A61K31/472(2006.01)i;A61K31/55(2006.01)i;A61K31/47(2006.01)i;A61K31/538(2006.01)i;A61K31/553(2006.01)i;A61P25/00(2006.01)i

According to International Patent Classification (IPC) or to both national classification and IPC

**B. FIELDS SEARCHED**

Minimum documentation searched (classification system followed by classification symbols)

C07D A61K A61P

Documentation searched other than minimum documentation to the extent that such documents are included in the fields searched

Electronic data base consulted during the international search (name of data base and, where practicable, search terms used)

CNABS; VEN; CNTXT; WOTXT; EPTXT; USTXT; CNKI; CJFD; WPABSC; VCN; STN; 万方; 读秀; ISI: 上海挚盟医药科技有限公司, 梁波, 刘刚, 江兆建, 黄孟炜, 陈焕明, 钾通道, 中枢神经系统, 四氢, 苯氮杂卓, 四氢异喹啉, potassium channel?, peripheral nervous system, 式I化合物的结构检索 structural search for compounds of formula I

**C. DOCUMENTS CONSIDERED TO BE RELEVANT**

| Category* | Citation of document, with indication, where appropriate, of the relevant passages | Relevant to claim No. |
|---|---|---|
| PX | CN 114057641 A (SHANGHAI ZHIMENG PHARMACEUTICAL TECHNOLOGY CO., LTD.) 18 February 2022 (2022-02-18)<br>abstract, and claims 1-10 | 1-10 |
| X | CN 112010808 A (SHANGHAI ZHIMENG PHARMACEUTICAL TECHNOLOGY CO., LTD.) 01 December 2020 (2020-12-01)<br>description, paragraphs [0006]-[0044], [0050]-[0055], and [0260] | 1-10 |
| X | US 2015284366 A1 (GAWECO, A. et al.) 08 October 2015 (2015-10-08)<br>description, paragraphs [0139]-[0142] | 1, 2 |
| X | CN 103508960 A (SHANGHAI SIMCERE PHARMACEUTICAL RESEARCH CO., LTD. et al.) 15 January 2014 (2014-01-15)<br>abstract, and claims 6-10 | 1, 2, 8-10 |
| A | US 2008146661 A1 (VALEANT PHARMACEUTICALS NORTH AMERICA) 19 June 2008 (2008-06-19)<br>entire document | 1-10 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **17 February 2023** | **28 April 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| Facsimile No. **(86-10)62019451** | Telephone No. |

Form PCT/ISA/210 (second sheet) (July 2022)

**INTERNATIONAL SEARCH REPORT**
**Information on patent family members**

International application No.
**PCT/CN2023/073185**

| Patent document cited in search report | | | Publication date (day/month/year) | Patent family member(s) | | | Publication date (day/month/year) |
|---|---|---|---|---|---|---|---|
| CN | 114057641 | A | 18 February 2022 | CA | 3163938 | A1 | 10 February 2022 |
| | | | | WO | 2022028548 | A1 | 10 February 2022 |
| | | | | AU | 2021319662 | A1 | 14 July 2022 |
| | | | | CN | 114845995 | A | 02 August 2022 |
| | | | | KR | 20220116466 | A | 23 August 2022 |
| | | | | EP | 4071138 | A1 | 12 October 2022 |
| | | | | IL | 294637 | A | 01 September 2022 |
| | | | | IN | 202217038774A | A | 18 November 2022 |
| | | | | BR | 112022013643 | A3 | 13 December 2022 |
| CN | 112010808 | A | 01 December 2020 | WO | 2020238917 | A1 | 03 December 2020 |
| | | | | US | 2022227713 | A1 | 21 July 2022 |
| | | | | CN | 112010808 | B | 30 November 2021 |
| | | | | CN | 113767091 | A | 07 December 2021 |
| | | | | KR | 20220016921 | A | 10 February 2022 |
| | | | | EP | 3978478 | A1 | 06 April 2022 |
| | | | | JP | 2022534533 | A | 01 August 2022 |
| | | | | HK | 40057653 | A0 | 14 April 2022 |
| | | | | US | 2022227713 | A1 | 21 July 2022 |
| US | 2015284366 | A1 | 08 October 2015 | US | 9321750 | B2 | 26 April 2016 |
| | | | | WO | 2013159095 | A1 | 24 October 2013 |
| | | | | EP | 2844247 | A1 | 11 March 2015 |
| | | | | EP | 2844247 | A4 | 25 November 2015 |
| CN | 103508960 | A | 15 January 2014 | WO | 2014000694 | A1 | 03 January 2014 |
| | | | | CN | 103508960 | B | 12 December 2017 |
| US | 2008146661 | A1 | 19 June 2008 | US | 7960436 | B2 | 14 June 2011 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **SAMBROOK et al.** Molecular Cloning: A Laboratory Manual. Cold Spring Harbor Laboratory Press, 1989
- **ZHAOBING GAO et al.** *Journal of Biological Chemistry*, 2010, vol. 285 (36), 28322-28332